(19) Europäisches Patentamt
European Patent Office
Office européen des brevets

(11) **EP 4 596 029 B1**

(12) **EUROPÄISCHE PATENTSCHRIFT**

(45) Veröffentlichungstag und Bekanntmachung des Hinweises auf die Patenterteilung:
**03.06.2026   Patentblatt 2026/23**

(21) Anmeldenummer: **24186391.9**

(22) Anmeldetag: **03.07.2024**

(51) Internationale Patentklassifikation (IPC):
**A61N 1/372** (2006.01)      **A61N 1/375** (2006.01)
**A61N 1/378** (2006.01)

(52) Gemeinsame Patentklassifikation (CPC):
**A61N 1/375; A61N 1/37229; A61N 1/3787; H02J 50/005;** A61B 2560/0219; A61N 1/37518; A61N 1/3756; H02J 50/90; H02J 2105/46

(54) **ELEKTRONISCHES IMPLANTAT**

ELECTRONIC IMPLANT

IMPLANT ÉLECTRONIQUE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **02.02.2024   DE 102024103021**
**11.04.2024   EP 24169789**
**18.04.2024   EP 24171165**

(43) Veröffentlichungstag der Anmeldung:
**06.08.2025   Patentblatt 2025/32**

(73) Patentinhaber:
• **Mehnert, Walter**
**85521 Ottobrunn (DE)**
• **Theil, Thomas**
**82340 Feldafing (DE)**
• **Reimann, Rainer**
**82386 Oberhausen (bei Peissenberg) (DE)**

(72) Erfinder:
• **Mehnert, Walter**
**85521 Ottobrunn (DE)**
• **Theil, Thomas**
**82340 Feldafing (DE)**
• **Reimann, Rainer**
**82386 Oberhausen (bei Peissenberg) (DE)**

(74) Vertreter: **Kilian Kilian & Partner mbB**
**Zielstattstraße 23a**
**81379 München (DE)**

(56) Entgegenhaltungen:
**EP-A1- 4 035 728        EP-A2- 3 756 726
US-A1- 2015 054 355    US-A1- 2017 202 467
US-A1- 2019 247 669    US-A1- 2020 001 095
US-A1- 2021 212 586**

**Beschreibung**

Die Erfindung betrifft ein elektronisches Implantat zur Implantation in einen Körper eines Lebewesens und zur Überwachung einer Körperfunktion. Insbesondere betrifft die Erfindung einen elektronischen Schrittmacher, der bestimmungsgemäß in/an das menschliche Herz implantiert wird.

Aus der Druckschrift US 2021/0212586 A1 ist ein Herzschrittmacher bekannt, der kontaktlos wieder aufgeladen werden kann. Der Schrittmacher weist eine Spule auf, die auf eine Ferritfolie geklebt ist. Insoweit bildet die Spule weder eine Luftspule noch eine Spule mit Kern. Die Fähigkeiten der Spule, ein zur Aufladung erzeugtes Magnetfeld zu sammeln, sind bei einer solchen Konstruktion beschränkt. Die flache Ausbildung der Spule führt zudem zu einem starken Gegenfeld und begrenzt damit den entnehmbaren Strom vorzeitig.

Der Schrittmacher ist konstruktiv so aufgebaut, dass er, wenn er am Herzen verankert ist, die Spule eine bestimmte Ausrichtung einnimmt, nämlich parallel zur Hautoberfläche des Trägers des Schrittmachers. Diese Ausrichtung ist notwendig, damit eine auf der Hautoberfläche angeordnete Ladespule zu der Spule des Schrittmachers ausgerichtet werden kann. Kommt es auch nur zu geringen Abweichungen zwischen Spule des Schrittmachers und der Ladespule sinkt der Ladestrom rapide ab. Außerdem muss die Ladespule auf der Hautoberfläche ein sehr starkes magnetisches Wechselfeld erzeugen, damit ein für die Aufladung adäquates Feld überhaupt die Spule in dem Schrittmacher erreicht.

Der Umstand, dass der Schrittmacher in einer bestimmten Weise verankert werden muss, ist eine extreme medizinische Bürde für den implantierenden Arzt. Sollte die Verankerung so misslich erfolgen, dass die Ausrichtung der Spule stark von der Soll-Ausrichtung abweicht, kann das dazu führen, dass der implantierte Schrittmacher gar nicht mehr geladen werden kann. Dokument US 2017/202467 offenbart ebenfalls eine ähnliche Vorrichtung.

Vor obigem Hintergrund ist es Aufgabe der Erfindung, ein Implantat zu schaffen, das räumlich beliebig implantierbar ist, extrem kurze Ladezeiten ermöglicht und gleichzeitig kompakt aufgebaut ist. Zumindest ist es Aufgabe der Erfindung, ein alternatives Implantat zu schaffen.

Diese Aufgabe(n) löst ein Implantat gemäß Patentanspruch 1. Bevorzugte Ausführungsformen sind Gegenstand der Unteransprüche.

*Das elektronische Implantat zur Implantation in einen Körper eines Lebewesens und zur Überwachung einer Körperfunktion, insbesondere der Schrittmacher zur Überwachung und Steuerung der Körperfunktion, beinhaltet:*

*einen Elektrodenabschnitt, der bestimmungsgemäß an einem Körperabschnitt zu befestigen oder anzuordnen ist; und*

*ein Gehäuse, das ein Volumen $V_G$ im Bereich von $0,5 \leq V_G \leq 4\ cm^3$, bevorzugt $\leq 2cm^3$, umfasst und das folgende Komponenten des elektronischen Implantates aufnimmt:*

*(i) eine mit dem Elektrodenabschnitt verbundene Elektronik, die eingerichtet ist, zumindest die Körperfunktion über den Elektrodenabschnitt zu überwachen;*

*(ii) einen Energiespeicher zur Langzeitversorgung der Elektronik mit elektrischer Energie, der nach Entladung mit elektrischer Energie wieder aufgeladen werden kann; und*

*(iii) einen mit dem Energiespeicher elektrisch verbundenen Energieempfangsabschnitt, der derart eingerichtet ist, dass er Energie kontaktlos empfangen und an den Energiespeicher zum Wiederaufladen des Energiespeichers abgeben kann; wobei*

*(I) der Energieempfangsabschnitt zumindest eine Spule, die sich entlang einer Spulenachse erstreckt und eingerichtet ist, die Energie zu empfangen und an den Energiespeicher abzugeben, wenn sie von einem magnetischen Wechselfeld, das ein externes Ladegerät erzeugt, durchsetzt wird, wobei die Spule eine Luftspule ist oder einen magnetisch leitenden Kern aus einem/mehreren Teilen, der sich in der Spule befindet und entlang der Spulenachse verläuft, beinhaltet, wobei*

*a) der Kern entlang der Spulenachse verläuft und über Enden der Spule hinaus nicht vorsteht, oder*

*b) der Kern entlang der Spulenachse verläuft und zur Ausbildung eines jeweiligen Feldkollektors über mindestens ein Ende, bevorzugt beide Enden, der Spule hinaus vorsteht, ohne dass sich ein Querschnitt/Längsdurchmesser des Kerns vergrößert, oder*

*c) der Kern entlang der Spulenachse verläuft und zur Ausbildung eines Feldkollektors über mindestens ein Ende der Spule hinaus vorsteht, wobei sich ein Querschnitt/Längsdurchmesser des Kerns vergrößert, und*

*(II) die Spule, wenn sie von dem magnetischen Wechselfeld durchsetzt wird, einen durch einen Gleichrichter gleichgerichteten Ladestrom von bevorzugt maximal 2A erzeugt, der dem Energiespeicher zum Wieder-*

*aufladen zugeführt wird;*

*(III) der Energieempfangsabschnitt eine senkrecht zur Spulenachse stehende Magnetfeldeinfangfläche $A_0$ mit $A_0 < =2{,}5*10^{-3}m^2$ besitzt, die durch $A_0 = \Phi_{SM}/B_0$ definiert ist, wobei $\Phi_{SM}$ der magnetische Fluss ist, der eine in Richtung der Spulenachse liegende magnetische Längsmitte bzw. eine Querschnittsfläche an einem Ort innerhalb der Spule als Maximum durchsetzt, und $B_0$ die externe, mittlere Flussdichte des magnetischen Wechselfeldes über der Magnetfeldeinfangfläche $A_0$ ist; und*

*(IV) die Elektronik eingerichtet ist, Informationen -zur automatischen räumlichen Anpassung einer Ausrichtung des Vektors des externen magnetischen Wechselfeldes an die Spulenachse- zu liefern.*

*Erfindungsgemäß ist die Elektronik gemäß (IV) eingerichtet, Informationen zur automatischen Korrektur einer Abweichung zwischen einer räumlichen Ausrichtung eines Vektors eines inneren magnetischen Wechselfeldes einer Ladespule eines Ladegerätes und der Spulenachse des Implantates zum Beispiel an das Ladegerät zu liefern, wodurch das Implantat in einer beliebigen räumlichen Orientierung implantierbar ist.*

*Das elektronische Implantat ist bevorzugt so aufgebaut, dass der Ort des maximalen magnetischen Flusses, der der magnetischen Längsmitte entspricht, die Längsmitte der Spule ist.*

Dies gilt für den Fall, dass der Energieempfangsabschnitt spiegelsymmetrisch aufgebaut ist.

Das Implantat ist beispielsweise ein Herzschrittmacher, ein Hirnschrittmacher, ein Organschrittmacher oder eine Analyseeinheit. Letztere Analyseeinheit ist beispielsweise so aufgebaut, dass sie kontinuierlich oder in bestimmten Zeitabständen Parameter, wie beispielsweise Blutdruck und/oder Blutwerte ermittelt und/oder ein Kardiogramm aufzeichnet. Ist das Implantat der genannte Herzschrittmacher, ist bevorzugt vorgesehen, vom Körper für das Herz gelieferte (körpereigene) Steuerungsimpulse zu reparieren, d.h. voll auszubilden, oder fehlende körpereigene Steuerungsimpulse zu ersetzen. Besonders bevorzugt ist das Implantat ein Einkammer-Herzschrittmacher oder ein Teil eines Mehrkammer-Herzschrittmachernetzwerks, der/das im menschlichen Herz oder am menschlichen Herz sitzt bzw. dort implantiert ist. Das Herzschrittmachernetzwerk besitzt beispielsweise zwei oder drei über elektrische Signale verbundene Implantate, die jeweils in eine Herzkammer implantiert, dort verankert werden, und miteinander kommunizieren.

Der Elektrodenabschnitt beinhaltet in Abhängigkeit davon, welchen Zweck das Implantat übernimmt, eine bestimmte Anzahl von Elektroden, wobei eine der Elektroden als Masse fungiert.

Wenn das Implantat die Funktion eines der genannten Schrittmacher übernimmt, werden die Elektroden bestimmungsgemäß mit dem Körperabschnitt, beispielsweise Herz oder Gehirn, der zu stimulieren ist, verbunden oder liegen an oder in diesem an.

Allgemein können die genannten Elektroden beispielsweise Kabelelektroden sein. Insbesondere beinhaltet das Implantat in diesem Zusammenhang bevorzugt pro Kabelelektrode ein Kabel bestimmter Länge, das bestimmungsgemäß zu einem gewünschten Bereich des Körperabschnitts innerhalb des Körpers verlegt werden kann. An dem Ende des Kabels ist ein bevorzugt spiralförmiger Abschnitt zur Verankerung der Kabelelektrode in dem Bereich des Körperabschnittes ausgebildet.

Alternativ kann der Elektrodenabschnitt auch ohne Kabelelektrode(n) auskommen. In diesem Fall sind die genannten Elektroden auf einer Außenoberfläche des Implantats ausgebildet, wobei es so implantiert wird, dass die Elektroden jeweils an oder in einem Bereich des Körperabschnitts anliegen und/oder dort verankert werden können. Diese Ausgestaltung ist insbesondere dann vorteilhaft, wenn das Implantat der Herzschrittmacher oder Teil des Herzschrittmachernetzwerks ist, die jeweils vollständig in/an das Herz zu implantieren sind. Das Herzschrittmachernetzwerk beinhaltet ergo dann mehrere erfindungsgemäße Implantate mit entsprechenden Elektrodenabschnitten, die auf der Außenoberfläche der jeweiligen Einheiten freiliegen.

Weiterhin alternativ kann der Elektrodenabschnitt aus einer Kombination aus mindestens einer einzigen Kabelelektrode und aus mindestens einer einzigen auf der Außenoberfläche ausgebildeten Elektrode aufgebaut sein. In diesem Fall wird das Implantat bevorzugt an dem Körperabschnitt angeordnet, sodass die auf der Außenoberfläche gebildete Elektrode mit dem entsprechenden Bereich des Körperabschnitts in Kontakt kommt und/oder dort verankert ist. Die andere Elektrode, d.h. die Kabelelektrode, wird zu einem anderen Bereich des Körperabschnittes geführt und dort verankert bzw. befestigt.

Die Elektronik des erfindungsgemäßen Implantats ist eingerichtet, zumindest eine oder mehrere Körperfunktionen zu überwachen. Hierzu gehören beispielsweise die Funktionen der genannten Analyseeinheit, d. h. die Aufzeichnung von Daten, beispielsweise von einem Kardiogramm, Blutdruckwerten oder die Aufzeichnung von Blutwerten. Hardwaretechnisch umfasst die Elektronik zur Ausführung entsprechender Funktionen zum Beispiel eine Rechenschaltung mit entsprechendem Speicher.

Wenn das Implantat der genannte Herzschrittmacher oder Teil des Herzschrittmachernetzwerks ist, die jeweils in/an das menschliche Herz zu implantieren sind, ist die Elektronik eingerichtet, den Herzschlag zu überwachen und basierend hierauf zu erkennen, ob der Herzschlag gesteuert werden muss. Wenn dies der Fall ist, erzeugt die Elektronik einen Stimulationsimpuls, insbesondere einen Spannungsimpuls, oder im Extremfall einen Spannungsstoß und gibt diesen über den Elektrodenabschnitt an den Körperabschnitt ab.

Im Hinblick auf den Aufbau und Funktionen des Herzschrittmachernetzwerks wird auf die Ausführungen in der Patentanmeldung EP 3756726 A2 verwiesen. Insbesondere werden die Absätze [0011-0028] der EP 3756726 A2 durch Bezug mit aufgenommen.

Der Energiespeicher zur Langzeitversorgung des erfindungsgemäßen Implantats ist bevorzugt ein elektrochemischer Akkumulator, der wieder aufgeladen werden kann, insbesondere ein Lithium-Ionen Akkumulator. Der Energiespeicher ist bevorzugt derart dimensioniert, dass er das gesamte Implantat für eine Laufzeit von 0,5, 0,6, 0,7, 0,8, 0,9, 1,0, 1,1, 1,2, 1,3, 1,4, 1,5, 1,6, 1,7, 1,8, 1,9, 2,0 Jahren (Ladeintervallen) mit der elektrischen Energie versorgen kann, ohne dass eine Aufladung des Energiespeichers erforderlich ist. Beispielsweise hat der Energiespeicher eine Ladungskapazität von 200 As bis 400 As (Amperesekunden, Coulomb).

Der Energiespeicher kann eine Vielzahl von Energiespeichereinheiten aufweisen, die verteilt und getrennt voneinander an verschiedenen Positionen in dem Implantat angeordnet sind, wobei zumindest eine der oder jede der Energiespeichereinheiten bevorzugt eine elektrochemische Akkumulatoreinheit, insbesondere eine Lithium-Ionen Akkumulatoreinheit ist.

Bevorzugt beinhaltet der Energieempfangsabschnitt mindestens einen Gleichrichter und mindestens einen Glättungskondensator, die sich zwischen der Spule und dem Energiespeicher befinden. Die Spule gibt die empfangene Energie über den Gleichrichter an den Glättungskondensator ab. In diesem Zusammenhang wird der von der Spule abgegebene Lade(wechsel)strom durch den Gleichrichter gleichgerichtet und von dem Glättungskondensator dem Energiespeicher zugeführt.

Der Energieempfangsabschnitt ist eingerichtet, die Energie per Induktion zu empfangen, wobei er hierfür eine Spule beinhaltet, die von dem externen magnetischen Wechselfeld durchsetzt wird. Die Spule erzeugt in Abhängigkeit von der Änderung des durchsetzenden magnetischen Flusses die entsprechende Ladespannung und über den Gleichrichter einen entsprechenden Ladestromfluss, der zum Wiederaufladen des Energiespeichers dient. Die Ladespannung ist anders ausgedrückt von Frequenz und Amplitude des Magnetflusses des magnetischen Wechselfeldes proportional abhängig.

Insbesondere ist der Kern gemäß a) und/oder der Feldkollektor gemäß b) oder c) kein Element, wie bspw. ein Wieganddraht/Impulsdraht, der bei einer Magnetfeldänderung bestimmter Amplitude einen großen Barkhausen-Sprung, in Form einer über den Draht laufenden Blochwand, zeigt und deshalb unabhängig von der Frequenz des magnetischen Wechselfeldes Impulse gleicher Höhe in der Spule induziert. Im Allgemeinen besitzt das Material des Kerns gemäß a) als Magnetflussleiter und das Material des Feldkollektors gemäß b) oder c) unregelmäßig magnetisch ausgerichtete Domänen.

Das Gehäuse nimmt die genannten Komponenten, d.h. die Elektronik, den Energiespeicher und den Energieempfangsabschnitt, in seinem Innenraum auf und schließt diese bevorzugt hermetisch ein. Das umfasste Volumen $V_G$ des Gehäuses beträgt vorzugsweise maximal $1,5 \, cm^3$, $2 \, cm^3$, $3 \, cm^3$ oder $4 \, cm^3$ wobei $1 \, cm^3$ bevorzugt ausgenommen ist. Der Elektrodenabschnitt ist, wie oben bereits erläutert, aufgebaut.

Das erfindungsgemäße Implantat ist damit insofern ein autonom arbeitendes Implantat, das seine Funktionen eigenständig ausführt, ohne dass hierfür eine Interaktion mit einer sich außerhalb des Körpers befindenden Steuerungseinheit erforderlich ist.

*Das Implantat ist erfindungsgemäß so aufgebaut, dass der Energieempfangsabschnitt eine senkrecht zur Spulenachse stehende Magnetfeldeinfangfläche $A_0$ mit $A_0 < = 2,5 * 10^{-3} m^2$ besitzt, die durch $A_0 = \Phi_{SM}/B_0$ definiert ist, wobei $\Phi_{SM}$ der magnetische Fluss ist, der eine in Richtung der Spulenachse liegende magnetische Längsmitte, die einer Querschnittsfläche der Spule an einem bestimmten Ort innerhalb der Spule entspricht, als Maximum durchsetzt, und $B_0$ die externe, mittlere Flussdichte über der Magnetfeldeinfangfläche $A_0$ ist; und*
*die Spule derart ausgebildet ist, dass sie bei Vorliegen des magnetischen Wechselfeldes den Ladestrom bevorzugt mit einer Stärke in einem Bereich von 20mA bis 2A erzeugt.*

Aus dem Bereich des Ladestromes ausgenommen sind bevorzugt Werte von ca. 200mA.

Bestimmungsgemäß wird das externe elektromagnetische Wechselfeld ($B_0$) bevorzugt so erzeugt, dass es in Richtung der Spulenachse ausgerichtet ist, d. h. der B-Vektor in die Richtung der Spulenachse weist.

Der Kern gemäß a) und/oder Feldkollektor gemäß b) oder c) sorgt dafür, dass das elektromagnetische Wechselfeld über eine größere Magnetfeldeinfangfläche (Feldsammelfläche) $A_0$ verstärkt in den Kern geleitet wird. Mit anderen Worten sorgt der Feldkollektor dafür, dass die magnetische Flussdichte innerhalb der Spule - $n*B_0$ - stark ansteigt.

Die Magnetfeldeinfangfläche $A_0$ befindet sich entlang der Spulenachse in einem bestimmten Abstand von dem Ende des Kerns gemäß a) oder dem Feldkollektor gemäß b) oder c) und verläuft senkrecht zur Spulenachse. Sie ist größer als der Kern gemäß a) oder der Feldkollektor gemäß b) oder c). Im Falle der Luftspule befindet sich die Magnetfeldeinfangfläche in der Längsmitte der Spule.

Diejenigen magnetischen Feldlinien, die die Magnetfeldeinfangfläche $A_0$ dursetzen, treten in die Spule ein (bspw. über den Feldkollektor gemäß b) oder c), und/oder den Kern gemäß a), b) oder c)) und durchlaufen die in Richtung der Spulenachse liegende Längsmitte der Spule.

Die Magnetfeldeinfangfläche $A_0$ ergibt sich aus dem maximalen magnetischen Fluss $\Phi_{SM}$ in der magnetischen Längsmitte bzw. durch eine Querschnittsfläche der Spule an einem bestimmten Ort der Spulenachse und der externen

Flussdichte $B_0$ des externen elektromagnetischen Wechselfeldes über $A_0$ nach der Beziehung $A_0 = \dfrac{\Phi SM}{B0}$ . Bei einem

symmetrischen Aufbau, insbesondere spiegelsymmetrischen Aufbau, des Energieempfangsabschnittes fallen die magnetische Längsmitte, die dem Ort der Querschnittsfläche der Spule, den der maximale magnetische Fluss durchsetzt, entspricht, und die (geometrische) Längsmitte der Spule zusammen.

Für den Kern und Feldkollektor gemäß b) oder c) gilt:
Bezeichnet man die in Richtung der Spulenachse weisende Länge des Kerns mit $I_k$, die in Richtung der Spulenachse weisende Länge des Feldkollektors mit $I_{FK}$ sowie den senkrecht zur Spulenachse verlaufenden Durchmesser des Feldkollektors mit $D_{FK}$, gilt konstruktiv in Näherung unter der Annahme kreisförmiger senkrecht zur Spulenachse verlaufender Querschnitte des Kerns und Feldkollektors

$$(I_K + 2I_{FK} + D_{FK})^2 * PI/8 < A_0 < (I_K + 2I_{FK} + D_{FK})^2 * PI/4.$$

Ein Ladegerät erzeugt bevorzugt das externe elektromagnetische Wechselfeld, bevorzugt mit einer magnetischen Flussdichte von $B_0$ = 0,02mT, 0,04mT, 0,1mT, 0,2mT, 0,5mT, 1mT, 2mT, 3mT, 4mT, 5mT, 6mT, 7mT, 8mT, 9mT, 10mT, 11mT, 12mT, 13mT, 14mT, 15mT, 16mT, 17mT, 18mT, 19mT, oder 20mT, und einer Frequenz f = 0,5kHz, 1 kHz, 2kHz, 3kHz, 4kHz, 5kHz, 6kHz, 7kHz, 8kHz, 9kHz, 10kHz, 11kHz, 12kHz,13kHz, 14kHz, 15kHz, 16kHz, 17kHz, 18kHz, 19kHz, 20kHz, oder f > 20kHz, oder ab f = 21kHz in 1 kHz-Schritten bis maximal 1,5 MHz. Die magnetische Flussdichte $B_0$ liegt insbesondere bestimmungsgemäß in einem großen räumlichen Bereich des implantierten Implantates konstant und homogen vor, beispielsweise im Herzbereich eines Menschen, an dem sich das kleine Implantat in der Funktion des Herzschrittmachers am/im Herz befindet.

Durch den/die Feldkollektoren gemäß b) oder c) plus Kern findet eine Verstärkung n der magnetischen Flussdichte innerhalb der Spule statt, wobei - verglichen mit dem Fall, dass kein Feldkollektor vorgesehen ist, - n >= 50, 60, 70, 80, 90, 100, 110, 120, 130, 140, 150, 160, 170, 180, 190, 200 ist. Damit ergibt sich maximal (Verstärkung 200) bei einer in der Umgebung der Spule räumlich homogenen magnetischen Flussdichte von beispielsweise $B_0$=1mT eine innerhalb des Kerns herrschende Kernflussdichte $B_K$ von 0,2T. Resultierende Werte der Spannung/des Stroms sind ausreichend, dem Energiespeicher ausreichend Energie zum Wiederaufladen zuzuführen, selbst wenn die Frequenz f des erzeugten magnetischen Wechselfeldes in den genannten niedrigen Bereichen liegt, beispielsweise bei 20kHz.

*Bevorzugt ist das elektronische Implantat derart ausgebildet, dass*

*der Energieempfangsabschnitt einen Gleichrichter beinhaltet,*
*die Spule eingerichtet ist, wenn sie von dem magnetischen Wechselfeld durchsetzt wird, einen durch den Gleichrichter gleichgerichteten Ladestrom zu erzeugen, der dem Energiespeicher zum Wiederaufladen zugeführt wird, und die Spule derart ausgebildet ist, dass sie bei Vorliegen des magnetischen Wechselfeldes den Ladestrom mit der Stärke in einem Bereich von 20mA bis 2A erzeugt.*

*Bevorzugt ist die Spule (6) derart ausgebildet, dass sie bei Vorliegen des magnetischen Wechselfeldes mit einer Frequenz in einem Bereich von 0,5 kHz bis 1,5 MHz und des magnetischen Flusses $\Phi_{SM}$ in einem Bereich von $1*10^{-9}$ Vs bis $5*10^{-5}$ Vs, den Ladestrom mit der Stärke in einem Bereich von 20mA bis 2A erzeugt.*

Der Wert $\Phi_{SM}$ = $1*10^{-9}$ Vs bezieht sich auf die minimal mögliche Fläche von $A_0$ bei minimalem $B_0$ = 0,02mT und der Wert $\Phi_{SM}$ = $5*10^{-5}$ Vs bezieht sich auf die maximale Fläche $A_0$ =$2,5*10^{-3}m^2$ bei maximalem $B_0$ = 20mT.

Die Spule besitzt beispielsweise W Windungen, wobei bevorzugt W = 10, 20, 30, 40, 50, 100, 200, 300, 400, 500, 600, oder 700, oder 800 ist. W ist maximal W=1000, wobei die genannten niedrigeren Werte wesentlich bevorzugter sind. Besonders bevorzugt ist W $\leq$ 50. Die durch die W Windungen gebildete Wicklung kann mehrschichtig oder bevorzugt einschichtig sein. Der die W Windungen bildende Metalldraht ist beispielsweise aus Kupfer oder bevorzugt aus dem leichteren Metall Aluminium und hat einen Drahtdurchmesser von beispielsweise 60$\mu$m, 70$\mu$m, 80$\mu$m, 90$\mu$m, 100$\mu$m, 150 $\mu$m, 200 $\mu$m, bis 700 $\mu$m und einen kreis- oder rechteckigen Querschnitt. Eine Länge der Spule 6 entspricht bevorzugt der des Kerns 7, sodass Enden der Spule bevorzugt Enden des Kerns entsprechen.

Aus der Windungsanzahl W, die in die Induktivität L der Spule quadratisch eingeht, ergibt sich der Wechselstromwiderstand der Spule zu $\omega$L, sowie aus Länge und Querschnitt des die Spule bildenden Metalldrahtes der entsprechende ohmsche Widerstand.

Durchläuft das magnetische Wechselfeld die Magnetfeldeinfangfläche $A_0$ des Energieempfangsabschnittes, tritt es folglich in die Spule ein und durchläuft damit die Spule, sodass die Spule den Ladestrom erzeugt.

Die Spule erzeugt aufgrund des entnommenen Ladestroms in dem genannten Bereich durch Selbstinduktion ein Gegenfeld innerhalb der Spule, das das die Spule durchsetzende externe Feld schwächt. Aus der Differenz zwischen externem Feld und Gegenfeld resultiert das den Ladestrom treibende Nutzfeld.

Die Stärke des Gegenfeldes ist proportional dem Produkt aus Ladestromstärke und Windungszahl, d.h. $I_{GL}$*W. Insoweit ist es wünschenswert, die Windungszahl gering zu halten. Dies führt aber zu dem gegenläufigen Effekt, dass das Nutzfeld eine geringere Spannung in der Spule induziert (EMK). Die induzierte Spannung bzw. die EMK entspricht der frequenzabhängigen Änderung des magnetischen Flusses $\Phi$ multipliziert mit der Windungszahl W (EMK=-W*d$\Phi$/dt).

Vor diesem Hintergrund ist bei Minimierung des Implantates die Windungszahl W der Spule für eine der oben angegebenen Frequenzen des magnetischen Wechselfeldes so gewählt, dass die durch das bestimmungsgemäße Nutzfeld in der Spule induzierte Spannung zwar für das Laden des Energiespeichers ausreichend hoch ist, aber gleichzeitig bspw. wegen des Gegenfeldes möglichst niedrig ist, und der Ladestrom in dem genannten Bereich zwischen 20mA und 2A liegt.

Lässt sich das für eine bestimmte Grenzfrequenz nicht realisieren, weil die durch das Nutzfeld induzierte Spannung für das Laden des Energiespeichers zu gering ist, kann bevorzugt entweder das externe magnetische Feld in seiner Stärke (B-Feld) oder durch die Konstruktion von Luftspule oder Kern gemäß a) und/oder Feldkollektor gemäß b)/c) die Magnetfeldeinfangfläche $A_0$ erhöht werden.

Hohe Feldstärken über große Bereiche des externen magnetischen Wechselfeldes lassen sich beispielsweise mit einem Ladegerät erzeugen, wie es in EP 4035728 A1 beschrieben ist.

Mittels eines Kompensationskondensators kann der Wechselstromwiderstand der Spule im Resonanz- oder Teilresonanzbetrieb so kompensiert werden, dass der Ladestrom in dem genannten Bereich zwischen 20mA und 2A liegt.

Der Kompensationskondensator ist ganz besonders bevorzugt in seiner Kapazität so gewählt, dass der Schwingkreis aus Spule und Kompensationskondensator sich gerade nicht in Resonanz befindet, sondern die Frequenz des externen magnetischen Wechselfeldes bis 10% über oder unter der Resonanzfrequenz des Schwingkreises liegt.

Der Energiespeicher besitzt bevorzugt einen maximalen Ladungsinhalt von 200As bis 400 As (Coulomb), um eine Langzeitversorgung des Implantates zu gewährleisten.

Dadurch, dass der Energieempfangsabschnitt eingerichtet ist, den Ladestrom in dem Bereich von 20mA und 2A zu erzeugen, kann das Implantat den Energiespeicher innerhalb einer Ladezeit von einer Stunde und weniger, vorzugsweise ≤ 20 min oder 30 min, aufladen.

In Abhängigkeit davon wie groß die Summe aus dem/den Feldkollektor(en) und dem Kern in Richtung der Spulenachse ist, ändert sich die Verstärkung n der magnetischen Flussdichte innerhalb des Kerns.

*Bevorzugt ist der Feldkollektor bzw. die Feldkollektoren gemäß b) und/oder c) ein Teil des Kerns, insbesondere mit dem Kern monolithisch, aus demselben Material gebildet.* Der/Die Feldkollektor(en) können alternativ ein gegenüber dem Kern gesondertes Element sein.

*Das Implantat ist bevorzugt so aufgebaut, dass der Kern gemäß a) oder b) oder c) eine magnetisch leitende Vollwelle oder eine magnetisch leitende Hohlwelle ist.*

*Beispielsweise ist der Kern die magnetisch leitende Vollwelle und der Energiespeicher befindet sich in Richtung der Spulenachse neben der Vollwelle und innerhalb oder außerhalb der Spule.*

*Alternativ hierzu ist die Spule auf die Vollwelle gewickelt und der Energiespeicher befindet sich radial zur Spulenachse um die Spule herum.* Beispielsweise kann der Energiespeicher die Spule vollständig umlaufen oder beispielsweise einen Winkelbereich über 180°oder 270° einnehmen. Der verbleibende freie Platz bzw. Freiraum, der sich radial und parallel zur Spulenachse erstreckt, kann für andere Elemente des Implantates verwendet werden, beispielsweise für Teile der Elektronik.

*Weiterhin beispielsweise ist der Kern die magnetisch leitende Hohlwelle, auf die die Spule gewickelt ist, und der magnetisch nichtleitende Energiespeicher befindet sich innerhalb der Hohlwelle. Alternativ hierzu befindet sich der magnetisch leitende Energiespeicher in Richtung der Spulenachse neben der Hohlwelle und innerhalb oder außerhalb der Spule.*

*Weiterhin beispielsweise ist der Kern ein magnetisch leitendes Gehäuse des Energiespeichers, auf den die Spule gewickelt ist.*

*Der Energiespeicher kann ein magnetisch nichtleitendes Gehäuse aufweisen und trotzdem als Trägerkörper für die Spule dienen, womit die Spule die genannte Luftspule bildet.*

*Besonders bevorzugt ist/sind der Kern und/oder der/die Feldkollektor(en) gemäß b) und/oder c) aus einem Material mit einer hohen (materialspezifischen) relativen magnetischen Permeabilität und/oder einer möglichst hohen (materialspezifischen) Sättigungsflussdichte gebildet.*

Beispiele für das Material sind Ferrite, insbesondere weichmagnetische Ferrite, oder amorphe Metalle, wie beispielsweise SiFe, das auch unter dem Markennamen ARNON erhältlich ist, oder Mu-Metalle, wie beispielsweise NiFe-Legierungen.

Der Kern und/oder der/die Feldkollektor(en) ist/sind beispielsweise ein Vollmaterial oder ein Schichtaufbau aus einer Vielzahl von Schichten. Auch kann der Kern beispielsweise teilweise aus einem Vollmaterial und einem teilweisen Schichtaufbau gebildet sein.

Der Schichtaufbau ist bevorzugt aus einer Vielzahl dünner Schichten, wie beispielsweise dünner Folien oder dünner Bleche, gebildet, zwischen denen jeweils elektrisch isolierende Schichten angeordnet sind. Die elektrisch isolierenden Schichten können die dünnen Schichten stoffschlüssig miteinander verbinden.

Wenn der Kern den Schichtaufbau besitzt, haben die einzelnen Schichten eine Dicke von beispielsweise 0,015mm, ... , 0,025mm, ... , 0,035mm, ... , 0,050mm. Die elektrisch isolierenden Schichten können dieselben Dicken aufweisen oder dünner sein.

Insbesondere ist es bevorzugt, dass die genannten Elemente (Kern und/oder Feldkollektoren) aus einem Vollmaterial gebildet sind, wenn das Material der Isolator ist, und dass die genannten Elemente (Kern und/oder Feldkollektoren) den Schichtaufbau besitzen, wenn das Material zwar schlecht aber zu einem gewissen Grad elektrisch leitend ist.

*Bevorzugt ist das Implantat so aufgebaut, dass*

*zur Wiederaufladung des Energiespeichers bestimmungsgemäß im Bereich des implantierten Implantates das magnetische Wechselfeld mit einer Flussdichte $B_0$ zu erzeugen ist, wodurch eine entsprechende Ladespannung in der Spule induziert wird, die zu einem von der Spule abgegebenen und dem Energiespeicher mittelbar oder unmittelbar zugeführten Lade(wechsel)strom führt,*
*der Kern und/oder der/die Feldkollektor(en) aus dem Material gebildet ist/sind, das die hohe Sättigungsflussdichte aufweist, und*
*eine Geometrie des Kerns und/oder der/die Feldkollektor(en) derart gewählt ist, dass eine Kernflussdichte $B_K$, die sich im Kern der Spule aus der vervielfachten Flussdichte ($n*B_0$) vermindert um ein durch den Lade(wechsel)strom erzeugtes Gegenfeld ergibt, im Bereich, insbesondere plus/minus 1%, 2%, 3%, 4%, 5% oder 10%, der (materialspezifischen) Sättigungsflussdichte liegt.*

Die magnetische Flussdichte $B_0$ kann die bereits erwähnten Werte aufweisen, insbesondere im Bereich von $20\mu T$ bis 20mT. Diese magnetische Flussdichte $B_0$ führt zu einem relativ starken Magnetfluss des magnetischen Wechselfeldes innerhalb des Kerns, weil der/die Feldkollektor(en) (und der Kern) das Feld entsprechend stark bündeln. Erfindungsgemäß wird das Material bevorzugt so gewählt, dass das um das Gegenfeld geschwächte im Kern herrschende Feld nahe der Sättigungsflussdichte oder in dem genannten Bereich liegt.

Durch diese Ausgestaltung wird das extern erzeugte magnetische Wechselfeld hinsichtlich Baugröße des Kerns/Feldkollektor(en) optimal ausgenutzt.

Der Ladestrom wird von der Spule dem Energiespeicher bevorzugt über eine Ladeelektronik, die den mindestens den Gleichrichter und mindestens den Glättungskondensator aufweist, zugeführt.

Unter der an verschiedenen obigen Stellen erwähnten Sättigungsflussdichte ist der dem Material spezifische Flussdichtebereich gemeint, bei dem die entsprechende Magnetisierungskennlinie (B-H-Kennlinie) einen Knickbereich bzw. Übergangsbereich besitzt, unterhalb dessen die Magnetisierungskennlinie im Wesentlichen linear verläuft und oberhalb dessen die Magnetisierungskennlinie mit geringerer Steigung (nämlich mit $\mu_0$) verläuft. Bevorzugt ist unter der Sättigungsflussdichte diejenige Flussdichte gemeint, bei der - bei weiterer Erhöhung der Feldstärke H des wirkenden magnetischen Wechselfeldes - die Polarisation des Materials nicht mehr weiter ansteigt.

Wie im Vorhergehenden bereits erwähnt, ist die Ausrichtung des Implantats bei Aufladung des Energiespeichers bevorzugt so zu realisieren, dass das externe elektromagnetische Wechselfeld bzw. der entsprechende Feldvektor (B-Vektor) bevorzugt in Richtung der Spulenachse weist und die Spule durchsetzt. Dies führt zu bestmöglicher Induktion und Ladestromimpulsen.

Bevorzugt besitzt die Elektronik des Implantats noch eine Kommunikationseinheit, über die sie mit der Außenwelt (außerhalb des Körpers des Lebewesens) kommunizieren (senden und/oder empfangen) kann, insbesondere zur (i) Übertragung der Informationen zur automatischen Korrektur der Abweichung zwischen der räumlichen Ausrichtung des Vektors des inneren magnetischen Wechselfeldes der Ladespule des Ladegerätes und der Spulenachse des Implantates, und (ii) damit in Zusammenhang stehenden Signale, wie z.B. das Startsignal.

Des Weiteren kann die Kommunikationseinheit bevorzugt mit der Außenwelt kommunizieren zur Übertragung von Einstellungsdaten, Einstellungsbefehlen, Analysedaten und/oder Informationsdaten, die den Ladezustand des Energiespeichers betreffen.

Für die Speicherung der Daten zwischen den Kommunikationen ist bevorzugt ein Datenspeicher zum Beispiel zusammen mit der bereits genannten Rechenschaltung vorgesehen.

*Die Elektronik ist gemäß (IV) erfindungsgemäß eingerichtet, Informationen zur räumlichen Anpassung einer Ausrichtung des magnetischen Wechselfeldes an die Spulenachse zu liefern, wodurch das Implantat in einer beliebigen räumlichen Orientierung implantierbar ist.*

Beispielsweise ist die mit dem Elektrodenabschnitt verbundene Elektronik eingerichtet, die Informationen zu generieren zur bevorzugt automatischen - vorzugsweise parallelen - Ausrichtung der Spulenachse, d.h. des Feldvektors des Ladegerätes zur Spulenachse des Implantates.

Insbesondere ist gemäß Patentanspruch 1 die Elektronik gemäß (IV) eingerichtet, die Informationen zur automatischen Korrektur der Abweichung zwischen der räumlichen Ausrichtung des Vektors des durch das Ladegerät erzeugten magnetischen Wechselfeldes und der Spulenachse des Implantates zu generieren und bevorzugt über die Kommunikationseinheit an das Ladegerät zu senden, wodurch das Implantat beliebig implantierbar wird. Das magnetische Wechselfeld erzeugt beispielsweise eine Ladespule des Ladegerätes, innerhalb derer sich der Körper des Patienten bei Aufladung befindet.

*Erfindungsgemäß nach Patentanspruch 1 ist gemäß (IV) die Elektronik eingerichtet,*

*(i) ein Startsignal zum Starten der Anpassung der Ausrichtung des magnetischen Wechselfeldes an das Ladegerät über die Kommunikationseinheit zu senden oder über die Kommunikationseinheit von diesem zu empfangen, wobei das Ladegeräte daraufhin die Ausrichtung des magnetischen Wechselfelds gemäß einer Bewegungsfunktion verändert, und*

*(ii) anschließend über die Kommunikationseinheit Zeitinformationen, die angeben, wann der Ladestrom zu der Wiederaufladung geeignet war, beispielsweise an das Ladegerät, als die Informationen zur Korrektur auszugeben.*

Die Elektronik kann die Informationen bevorzugt durch Messen oder Verarbeiten generieren, wobei die Informationen beispielsweise Werte über Amplituden und/oder Gradienten von induzierter Spannung und/oder Ladestromstärke beinhalten.

Die generierten Informationen bilden damit die Basis, dass das Implantat an/in völlig beliebiger Position, d.h. Ort und räumlicher Ausrichtung, in den Körper des Lebewesens implantiert werden kann.

Insbesondere ist die Elektronik bevorzugt eingerichtet, den Gradienten und/oder die Amplitude der induzierten Ladespannung und/oder des durch die induzierte Ladespannung getriebenen Ladestroms zu detektieren, und hierauf basierend die Zeitinformationen zu generieren. Die Elektronik evaluiert insbesondere, ob der Ladestrom zur Wiederaufladung geeignet insbesondere maximal war, beispielsweise auf Basis des Gradienten und/oder der Amplitude des Ladestroms, wobei sie hierbei bevorzugt einen Ladungszustand des Akkumulators berücksichtigt.

Die Kommunikationseinheit versendet die Zeitinformationen an die Außenwelt, wobei eine übergeordnete, die Zeitinformationen empfangende Einheit, wie beispielsweise das Ladegerät (bevorzugt gemäß EP 4035728 A1), aus den Zeitinformationen auf die beliebige Lage und Ausrichtung des Implantates schließen kann. Mit Kenntnis der Lage und Ausrichtung des Implantates kann die übergeordnete Einheit, wie bspw. das Ladegerät den B-Feldvektor des magnetischen Wechselfeldes zur Optimierung der Aufladung entsprechend ausrichten.

Hervorzuheben ist hierbei, dass die anfängliche Ausrichtung des B-Feldvektors beliebige Richtungen im Raum einnehmen kann, weil immer eine anschließende Anpassung an die Lage und Ausrichtung des Implantates möglich ist. Auch folgt hieraus, dass bei Implantation des Implantates keine Beachtung der resultierenden Lage und Ausrichtung des Implantates geschenkt werden muss. Gleiches gilt für die Alternative nach Patentanspruch 6.

Die Bewegungsfunktion, gemäß der das Ladegerät die Ausrichtung des magnetischen Wechselfeldes, bspw. den B-Feldvektor, ändert, kann beliebig sein und bedarf auch keiner besonderen Startausrichtung des Feldes. Erforderlich ist lediglich, dass die Bewegungsfunktion eine Funktion in Abhängigkeit von der Zeit ist (f(t)), aus der das Ladegerät nach Durchlaufen der Bewegungsfunktion schließen kann, zu welchem Zeitpunkt das magnetische Wechselfeld welche Ausrichtung hatte.

Empfängt das Ladegerät nach Durchlaufen der Bewegungsfunktion nunmehr die Zeitinformationen von dem erfindungsgemäßen Implantat, kann es mit Hilfe der Bewegungsfunktion aus den Zeitinformationen die geeignete Ausrichtung ermitteln werden.

Das Ladegerät kann die im Vorhergehenden angesprochene Ausrichtung des Wechselfeldes gemäß folgenden Optionen vornehmen:

- Das Ladegerät kann eine Körperhalterung (bspw. Stuhl oder Liege), auf der sich der Körper befindet, bevorzugt um drei orthogonale Achsen drehen und/oder linear versetzen; und/oder
- Das Ladegerät kann eine Ladespule, die das magnetische Wechselfeld erzeugt, bevorzugt um zwei oder drei orthogonale Achsen drehen und/oder linear versetzen; und/oder
- Das Ladegerät kann das magnetische Wechselfeld, das bevorzugt durch eine Vielzahl von Ladespulen erzeugt werden, dadurch ausrichten, dass Betriebsparameter der Ladespulen verändert werden und sich individuelle magnetische Wechselfelder der einzelnen Ladespulen zu dem magnetischen Wechselfeld mit einer bestimmten Ausrichtung überlagern.

*Besonders bevorzugt ist (V) die Elektronik eingerichtet ist,*

*(i) einen Abschluss der räumlichen Anpassung der Ausrichtung des magnetischen Wechselfeldes zu erkennen oder von dem Ladegerät über die Kommunikationseinheit signalisiert zu bekommen, die Zeitinformationen zur Korrektur an das Ladegerät auszugeben, das anschließend die Endstellung zum Aufladen einnimmt.*

*Bevorzugt geben die Zeitinformationen zumindest einen Zeitpunkt oder einen Zeitbereich an, zu dem/in dem der Ladestrom zur Wiederaufladung maximal war.*

Die Zeitinformationen sind beispielsweise in einer Zeiteinheit (Sekunden, Hundertstel-Sekunden, oder Millisekunden) angegeben.

Allgemein kann das Implantat neben den Zeitinformationen auch zugehörige Stromstärken- und/oder Ladungsinformationen an das Ladegerät ausgeben bzw. über die Kommunikationseinheit übertragen.

Der Zeitpunkt ist insbesondere ein solcher Zeitpunkt, an dem der Ladestrom ein Maximum erreichte. Der Zeitbereich hingegen ist eine Zeitspanne, in der der Ladestrom ein Maximum durchließ. Insbesondere ist der Zeitbereich dadurch definiert, dass er den Zeitpunkt des maximalen Ladestroms und zeitlich davor und danach liegende Zeitpunkte, in denen der Ladestrom maximal x% darunterlag, beinhaltet, wobei x%=1%, 2%, 3%, 4%, 5%, 6%, 7%, 8%, 9%, 10% ist.

*Insbesondere bevorzugt geben die Zeitinformationen in Form von Zeitpunkten oder Zeitbereichen an, an denen/in denen der Ladestrom maximal war, wobei die Elektronik eingerichtet ist, (i) die Zeitpunkte oder Zeitbereiche dahingehend zu qualifizieren, ob der jeweilige maximale Ladestrom ein globales oder lokales Maximum darstellt, und (ii) zumindest den Zeitpunkt oder den Zeitbereich gemäß dem globalen Maximum, beispielsweise an das Ladegerät, als die Informationen zur Korrektur auszugeben.*

Wie bereits erwähnt evaluiert die Elektronik, ob der Ladestrom geeignet war bzw. maximal war, auf Basis des Gradienten und/oder der Amplitude des Ladestroms, wobei sie hierbei bevorzugt den Ladungszustand des Akkumulators berücksichtigt.

Besonders bevorzugt übermittelt das Implantat Zeitpunkte/Zeitbereiche verschiedener Maxima, insbesondere aller Maxima, an das Ladegerät. Das Ladegerät kann anschließend entscheiden, welche Ausrichtung des magnetischen Wechselfeldes es für die Wiederaufladung einstellt.

Bevorzugt ist das erfindungsgemäße Implantat ausgestaltet, dass die Elektronik die Stärke des Ladestroms in regelmäßigen oder unregelmäßigen Intervallen bestimmt und hierauf basierend die Zeitinformationen ermittelt und für das Ausgeben abspeichert.

Wie bereits erwähnt kann das Implantat hierbei zusätzlich die zugehörigen Stromstärken- und/oder Ladungsinformationen für das Ausgeben speichern. Diese zusätzlichen Stromstärken- und/oder Ladungsinformationen sind insbesondere dann vorteilhaft, wenn das Implantat die Zeitinformationen gemäß lokalem und globalen Maximum überträgt. Das Ladegerät kann nach Empfang der Zeitinformationen inklusive der Stromstärken-/Ladungsinformationen eine Entscheidung treffen, ob es das magnetische Wechselfeld gemäß dem lokalen oder globalen Maximum für das Wiederaufladen ausrichtet.

Bevorzugt speichert die Elektronik die Stärken des Ladestroms nicht über den gesamten Zeitraum vom Startsignal für die Anpassung bis Abschluss der Anpassung, sondern nur deren Maxima zur späteren Ausgabe mit der entsprechenden Zeitinformation.

*Alternativ und erfindungsgemäß nach Patentanspruch 6 ist gemäß (IV) die Elektronik eingerichtet, Informationen zur automatischen Korrektur einer Abweichung zwischen einer räumlichen Ausrichtung eines Vektors eines inneren magnetischen Wechselfeldes einer Ladespule des Ladegerätes und der Spulenachse des Implantates, zum Beispiel an das Ladegerät, bevorzugt über die Kommunikationseinheit zu liefern, wobei*

*die Elektronik einen Speicher besitzt, in dem mindestens ein Schwellenwert, der einer bestimmten Stärke des Ladestromes entspricht, gespeichert ist, und*
*die Elektronik eingerichtet ist, den Ladestrom mit dem Schwellenwert zu vergleichen, und, wenn der Ladestrom den Schwellenwert erreicht, zumindest diese Information zur automatischen Korrektur der räumlichen Ausrichtung der Spulenachse des Implantates an das Ladegerät in zeitlichem Zusammenhang, bevorzugt unmittelbar und unverzögert, zu liefern.*

Unter zeitlichem Zusammenhang ist zu verstehen, dass das Ladegerät, wenn es die Informationen zur automatischen Korrektur der räumlichen Ausrichtung empfängt, aus dem Zeitpunkt des Empfanges die Ausrichtung des B-Vektors des magnetischen Wechselfeldes, die zu dem Erreichen des Schwellenwertes führte, ableiten kann.

Besonders bevorzugt erfolgt das Versenden der Information zur automatischen Korrektur der Ausrichtung unmittelbar und unverzögert, wobei hierunter zu verstehen ist, dass das Versenden nur durch die (hardwarebedingte) Verarbeitungsgeschwindigkeit der Elektronik verzögert ist. Bei Erhalt der Informationen durch das Ladegerät kann dieses aus dem Zeitpunkt des Empfanges, der quasi identisch ist mit dem Zeitpunkt, zu dem der Schwellenwert erreicht wird, die räumliche Ausrichtung des B-Vektors ableiten.

Besonders bevorzugt sind in dem Speicher eine Vielzahl von Schwellenwerten abgespeichert, wobei jeder einer unterschiedlichen bestimmten Stärke, z.B. des Ladestromes, entspricht.

*Wenn der Ladestrom, z.B. im Rahmen der Drehung des Ladegerätes um eine der Achsen eines, z.B. auf das Ladegerät bezogene Koordinatensystem, einen der Schwellenwerte erreicht, gibt die Elektronik zumindest diese Information zur Erfassung und Feststellung der momentanen räumlichen Ausrichtung der Spulenachse des Ladegerätes*

*bevorzugt unmittelbar und unverzögert aus in Hinblick auf die entsprechende Korrektur.* Die Ausgabe erfolgt wieder insbesondere über die Kommunikationseinheit.

Die Ausgabe der Informationen zur automatischen Korrektur der Ausrichtung erfolgt in dem zeitlichen Zusammenhang, insbesondere unmittelbar und unverzögert, bevorzugt aus Zuverlässigkeitsgründen n-mal, wobei bevorzugt n= 1, 2 oder 3 ist. Hiernach würde nach Drehung um beispielsweise eine Koordinatenachse die Ausgabe für den entsprechenden Schwellenwert erst dann wieder erfolgen, wenn z.B. das Ladegerät zur Drehung auf die nächste Koordinatenachse wechselt.

Falls der Ladestrom einen aus der Vielzahl von Schwellenwerten erreicht und vorher bereits einen darunterliegenden überschritten hatte, kann das Ladegerät die Information über den niedrigeren Schwellenwert bevorzugt verwerfen. Beispielsweise fährt das Ladegerät zwei oder drei zueinander orthogonale Bahnen ab und speichert die Zeitpunkte und damit die Stellungen, zu denen die höchsten Schwellenwerte erreicht wurden. Anschließend ist das Ladegerät in der Lage, aus den Zeitpunkten bzw. Stellungen die Lage des Implantates im Körper des Patienten zu ermitteln und seine Achse optimal parallel auszurichten.

Bevorzugt ist (V) die Elektronik des Implantates eingerichtet,

(i) einen Abschluss der räumlichen Anpassung der Ausrichtung des magnetischen Wechselfeldes zu erkennen oder von dem Ladegerät signalisiert zu bekommen, und
(ii) anschließend auf Basis des momentanen Ladestroms dem Ladegerät ein Feldänderungssignal zu senden, das dem Ladegerät signalisiert, Frequenz und/oder Amplitude des magnetischen Wechselfeldes zu erhöhen oder zu vermindern, wobei die Elektronik dem Ladegerät anschließend signalisiert, welche Auswirkungen die Änderungen der Frequenz und/oder Amplitude des magnetischen Wechselfeldes nach sich ziehen.

In diesem Zusammenhang geht das Implantat bevorzugt, gemäß beiden Varianten nach Patentanspruch 1 oder 6 und deren bevorzugten Ausgestaltungen, in einen Feinjustiermodus über, in dem es weiß, dass das Ladegerät seine räumliche Ausrichtung nicht mehr ändert, sondern Frequenz und/oder Amplitude des magnetischen Wechselfeldes. In diesem Feinjustiermodus sendet das Implantat beispielsweise Ladestrominformationen in festen Intervallen, sodass das Ladegerät Auswirkungen der Änderungen von Frequenz und/oder Amplitude des magnetischen Wechselfeldes interpretieren kann zur Kompensation der körperspezifischen Dämpfung des magnetischen Feldes und zur Erzielung einer festgelegten Amplitude des Ladestromes.

Besonders bevorzugt ist das erfindungsgemäße Implantat, insbesondere die bevorzugten Varianten des Energieempfangsabschnittes so ausgebildet und dimensioniert, dass bei paralleler Ausrichtung von B-Feldvektor und Spulenachse und bei einer magnetischen Flussdichte $B_0$ von 0,02mT bis 1mT des externen magnetischen Wechselfeldes sich bei genanntem maximalen $A_0$ ein mittlerer, magnetischer Fluss von $5*10^{-8}$ bis $2,5*10^{-6}$ Vs (Weber) im Kern einstellt, wobei der magnetische Fluss sich auf den unbelasteten Fall ohne Gegenfeld bezieht.

Für den Fall, dass der Wechselstromwiderstand ($\omega L$) der Spule den ohmschen Widerstand R der Spule deutlich übersteigt, ist, wie bereits erwähnt, bevorzugt, dass die Elektronik einen zusätzlichen Kompensationskondensator besitzt, und das magnetische Wechselfeld bei der Frequenz erzeugt wird, die sich aus den Werten der Spule, des Kompensationskondensators, des ohmschen Widerstandes und der Last ergibt, also im Wesentlichen der ohmsche Widerstand R die Größe/Stärke des Ladestroms begrenzt.

Letztendlich ist der Aufbau des Implantates bei einer vorbestimmten Baugröße des Implantates, die durch das Volumen des Gehäuses gegeben ist, z.B. zylinderförmiges Gehäuse, mit einer Spule mit den W Windungen und einem Windungsquerschnitt A und einem externen Magnetfeld $B_0$ bevorzugt in Bezug auf den erzielten Ladestrom optimiert. Im Fall von ($\omega L >> R$) gilt in erster Näherung, dass der Ladestrom proportional zu dem Verhältnis des Magnetflusses durch die Spule zur Induktivität der Spule ($\Phi/L$) ist, im Resonanzfall ($\Phi/R$). Deshalb ist die Summe der Abmessungen der Feldkollektoren und der Länge des Kerns in Richtung der Spulenachse mit dem Durchmesser der Feldkollektoren ein wichtiges Maß für die Höhe des entnehmbaren Ladestromes. Die genannten Parameter beeinflussen nämlich sowohl den Magnetfluss im Kern als auch die Induktivität der Spule.

Bei dem erfindungsgemäßen Implantat ist das konstruktive Verhältnis bevorzugt so realisiert, dass durch die Wahl der elektrischen Parameter der Ladestrom sein Maximum erreicht oder bis zu 10% darunterliegt. Hervorzuheben ist in Zusammenhang mit dem erzielten Ladestrom, dass der ohmsche Widerstand R der Spule durch geringe Windungszahl bei gleichzeitig hoher Feldstärke im Kern sehr gering gehalten werden kann. Der geringere ohmsche Widerstand erzeugt geringere Verluste und hat damit eine wesentlich geringere Wärmentwicklung zur Folge. Aufgrund der vorgesehenen Anordnung des Implantats im menschlichen Körper, z.B. Herz, Hirn, Gewebe, Gefäß oder Organ, ist dies ein ganz wesentlicher Faktor.

Aus den Erläuterungen des erfindungsgemäßen Implantates und seinen bevorzugten Merkmalen kann Folgendes abgeleitet werden:
Die beschriebene Konstruktion des Implantates, insbesondere des Energieempfangsabschnittes, eröffnet die signifikante Möglichkeit durch viele veränderbare Parameter ein Optimum für den jeweiligen Anwendungsfall des Implantates beispielsweise als Herzschrittmacher, Hirnschrittmacher, Organschrittmacher oder Analyseeinheit zu finden. Dieses Optimum lässt sich finden, indem das in der Spule resultierende Magnetfeld (Nutzfeld) maximiert wird, das Gewicht, die Verluste und insbesondere die Windungszahl W der Spule minimiert werden und das Volumen des Implantates in erster Näherung durch die Abmessungen der magnetischen Komponenten vorgegeben wird.

Bei einer gewünschten geringen Größe und Gewicht und einer autonomen Betriebszeit des Implantates von beispielsweise einem Jahr und einer Ladezeit von beispielsweise kleiner als 30 Minuten ist bei der erläuterten Konstruktion des Implantates, insbesondere des Energieempfangsabschnittes, eine Optimierung hinsichtlich des Volumens und Gewichts in erster Näherung durch ein möglichst großes externes durch die Medizin beschränktes Magnetfeld (< 1mT), in zweiter Näherung durch die bei der Aufladung auftretenden Verluste bzw. Erwärmung und der Feldkonzentration in der Spule oder im Kern gegeben.

Das erfindungsgemäße Implantat, insbesondere in seiner Ausgestaltung als akkubetriebener, autonomer Herzschrittmacher, ist universell einsetzbar und hinsichtlich Volumen und Gewicht minimiert. Hierdurch erfüllt es hohe Anforderungen und überwindet damit technologische Grenzen. Beispielsweise erlaubt die Konstruktion des Implantates mit folgenden hohen Anforderungen klarzukommen bzw. diese zu erfüllen:

## Externes magnetische Wechselfeld (B-Feld) ≤ 1mT;

Ladekapazität: 400As;
Ladeintervall > 1 Jahr,
Ladedauer < 1 oder 0,5 Stunde,
Volumen < 2 cm$^3$,
hier maximal Verlustleistung < 60 mW.

Generell gilt: je größer das externe, magnetische B-Feld, desto geringer die Ladezeit.

Das maximale externe Feld (wahrscheinlich ≤ 1mT) wird vom menschlichen Körper im Zusammenspiel mit der Einwirkdauer über die Verträglichkeit (medizinisch) festgelegt.

Im Grenzfall führen 400 As kurzzeitig ($10^3$ sec) zu einem Ladestrom von $I_{GI}$ = 0,4 A. Das aus diesem relativ großen Ladestrom resultierende und beachtliche Gegenfeld, das proportional $I_{GI}$ * W ist, bleibt durch geringe Windungszahlen von beispielsweise W≤ 50 und dem Umstand, dass die Magnetfeldeinfangfläche(n) im Verhältnis zu der geringen räumlichen Größe des Implantates ausreichend groß ist/sind trotz bzw. selbst bei geringem externen magnetischen Wechselfeld $B_0$, derart beherrschbar, dass das Nutzfeld (Differenz aus im Kern konzentrierten Magnetfeld und Gegenfeld) den Ladestrom treiben kann.

Hinzukommt, dass, bei dem geringen Volumen des Implantates und der Konstruktion aus Kern gemäß a) und Feldkollektor(en) gemäß b)/c) mit einem Kerndurchmesser von $d_k$ ≤ 2mm, die Wicklungsverluste (Kupfer- oder Aluminiumverluste) wegen kleiner Windungszahl und das Gewicht des Implantates gering sind. Der Umstand, dass bei dem erfindungsgemäßen Implantat das im Kern konzentrierte Magnetfeld ausreichend homogen durch den Kern verläuft und die Windungszahl W gering ist, kann die Spule bevorzugt einschichtig sein, was für einen hohen Wirkungsgrad und einer Gewichtsverminderung förderlich ist.

Die genannten Anforderungen und/oder die erläuterten Effekte können selbst dann erzielt werden, wenn das externe magnetische Wechselfeld mit hohen Frequenzen erzeugt wird. Der aufgrund der kleinen Windungszahl ohnehin geringe Wechselstromwiderstand kann durch den Kompensationskondensator und den Teilresonanz- oder Resonanzbetrieb weiter reduziert werden zur Optimierung des entnehmbaren Stromes.

Die vorstehenden Ausführungen gelten für die folgende Ausführungsform gleichermaßen.

Im Folgenden wird eine bevorzugte Ausführungsform unter Bezug auf die beigefügten Figuren erläutert.

**Figur 1A** zeigt eine bevorzugte Ausführungsform des erfindungsgemäßen Implantats, wobei die Darstellung lediglich schematischer Art ist;

**Figur 1B** zeigt eine schematische Schnittansicht eines Energieempfangsabschnitts des erfindungsgemäßen Implantats;

**Figur 1C** zeigt den Kern gemäß Figur 1B und einen entsprechenden Feldverlauf des zur Aufladung des Implantates erzeugten magnetischen Wechselfeldes mit der konstruktionsprägenden Fläche $A_0$;

**Figuren 2A bis 2C** zeigen weitere Ausgestaltungen des erfindungsgemäßen Implantates;

**Figur 3** zeigt eine schematische Schnittansicht eines alternativen Energieempfangsabschnitts des erfindungsgemäßen Implantats; und

**Figur 4** zeigt das Implantat 100 in beliebiger Position im Körper eines Menschen, wobei durch nur zwei Rotationsschritte die deckungsgleiche räumliche Lage mit der Achse der Spule des Ladegerätes erzwungen wird als Ausgangslage für eine optimale Aufladung des Energiespeichers des Implantats 100.

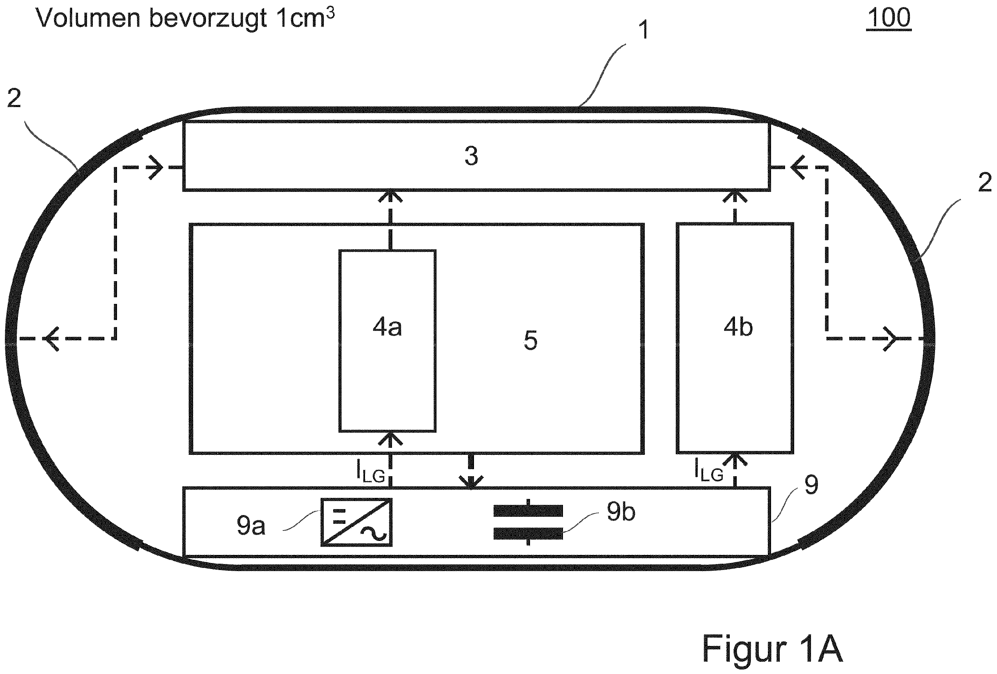

Die Figur 1A zeigt schematisch den Aufbau eines erfindungsgemäßen Implantats 100.

Das Implantat 100 wird bevorzugt vollständig in einen menschlichen Körper implantiert, wobei aufgrund der im Folgenden noch erläuterten Funktionen der Elektronik 3 eine sich ergebende räumliche Orientierung des Implantates 100 beliebig sein kann. Anders ausgedrückt kann der Arzt das Implantat implantieren, ohne Rücksicht nehmen zu müssen auf die resultierende Orientierung.

Das Implantat 100 ist beispielsweise ein Herzschrittmacher, ein Hirnschrittmacher, ein Organschrittmacher oder eine Analyseeinheit. Letztere Analyseeinheit ist beispielsweise so aufgebaut, dass sie kontinuierlich oder in bestimmten Zeitabständen Parameter, wie beispielsweise Blutdruck und/oder Blutwerte ermittelt. Besonders bevorzugt ist das Implantat ein Herzschrittmacher oder Herzschrittmachernetzwerk, der/das im menschlichen Herz oder am menschlichen Herz sitzt bzw. an diese Positionen zu implantieren ist.

Das Implantat 100 besitzt bevorzugt ein Gehäuse 1, das alle Elemente des Implantats 100 aufnimmt und bevorzugt hermetisch eingekapselt. Das Gehäuse 1 ist beispielsweise aus Titan oder Glas gebildet. Weiterhin alternativ kann das Gehäuse 1 aus einem körperverträglichen Kunststoff gebildet sein. Vorteilhaft an dem Kunststoff ist, dass das Gehäuse 1 gebildet werden kann, indem die darin aufgenommenen Komponenten mit dem Kunststoff umspritzt/umgossen werden.

Das Implantat 100 besitzt einen Elektrodenabschnitt mit Elektroden 2, der in Abhängigkeit davon, welchen Zweck das Implantat übernimmt bzw. welche Körperfunktion er überwachen/stimulieren soll, eine bestimmte Anzahl von Elektroden 2 aufweist. Die Elektroden 2 werden bestimmungsgemäß mit dem Körperabschnitt, beispielsweise Herz oder Gehirn, der zu überwachen und/oder zu stimulieren ist, verbunden oder liegen an diesem an.

Die Elektroden 2 können beispielsweise an ihren Enden spiralförmige Abschnitte, die in den Körperabschnitt gedreht und so verankert werden, aufweisen. Eine der Elektroden und/oder das Gehäuse, wenn elektrisch leitend, können als Masseelektrode dienen.

Allgemein kann es sich bei dem erfindungsgemäßen elektronischen Schrittmacher oder dem Herzschrittmachernetzwerk um einen Herzschrittmacher gemäß einem beliebigen NBG-Code handeln.

Allgemein können die genannten Elektroden beispielsweise Kabelelektroden oder auf der Außenoberfläche freiliegende Elektrodenflächen sein.

In dem Gehäuse 1 aufgenommen sind zudem eine Elektronik 3, die eingerichtet ist, über die Elektroden 2 eine Körperfunktion zu überwachen und/oder zu stimulieren, und ein Energiespeicher mit einer Vielzahl von, bevorzugt zwei, Energiespeichereinheiten 4a, 4b, die die Elektronik 3 mit elektrischer Energie langzeitversorgen sowie eine Ladeelektronik 9.

Bevorzugt sind in der Ladeelektronik 9 ein Gleichrichter 9a und ein Kondensator 9b enthalten, die einen von der Spule 6 abgegebenen Lade(wechsel)strom $I_L$ gleichrichten und als $I_{LG}$ den Energiespeichereinheiten 4a, 4b zuführen, indem der Gleichrichter 9a den von der Spule 6 abgegebenen Lade(wechsel)strom $I_L$ gleichrichtet und dem Kondensator 9b zuführt, und der Kondensator 9b daraufhin den Strom $I_{LG}$ an die Energiespeichereinheiten 4a, 4b weitergibt.

Die Energiespeichereinheiten, d.h. die eine Energiespeichereinheit 4a und die bevorzugte, weitere Energiespeichereinheit 4b, sind bevorzugt jeweils wiederaufladbare, elektrochemische Akkumulatoren, beispielsweise Lithium-Ionen-Akkumulatoren, die das gesamte Implantat 100 für beispielsweise 0,5 bis 1,5 Jahre mit elektrischer Energie versorgen, bevor sie wieder aufgeladen werden müssen. Die Energiespeichereinheiten 4a, 4b dienen folglich zur Langzeitversorgung des Implantates 100.

Die Energiespeichereinheiten 4a, 4b können kontaktlos wieder aufgeladen werden, unter Ausnutzung von Induktion. Hierfür besitzt das Implantat 100 einen Energieempfangsabschnitt 5.

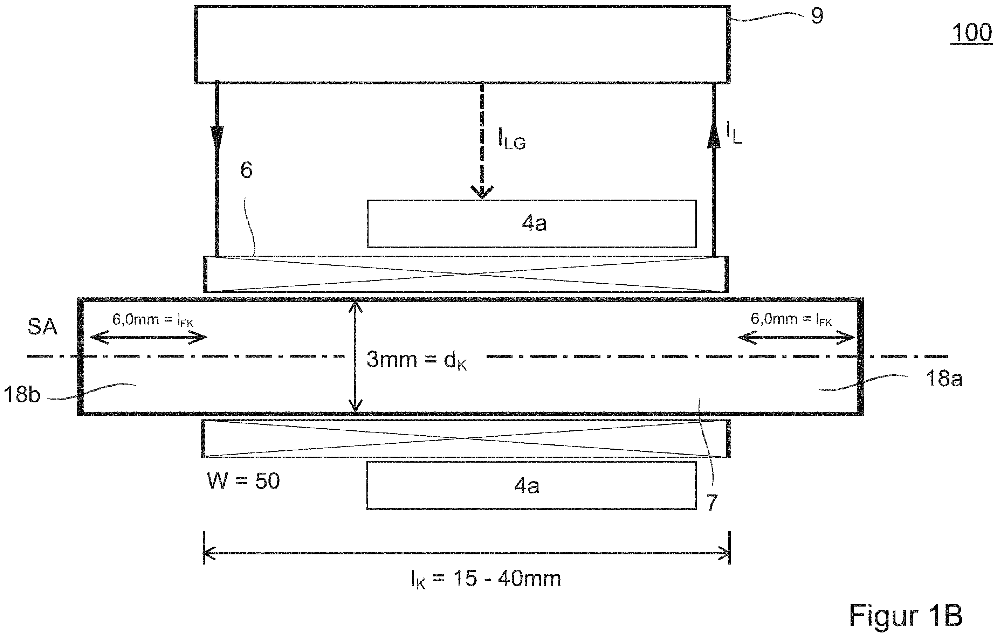

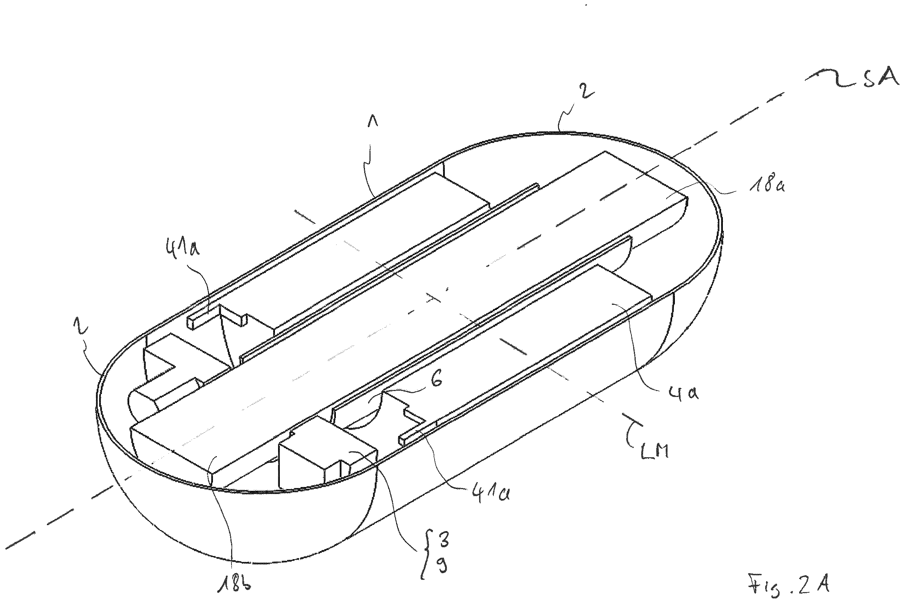

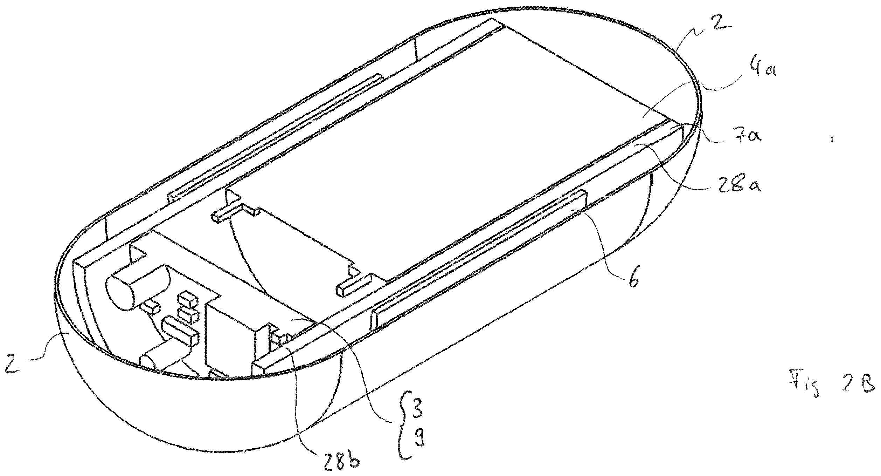

Die Energiespeichereinheit 4a befindet sich in einem Umfangsbereich (siehe Figur 1B oder Figur 2A) oder innerhalb eines Kerns 7 (siehe Figur 2B), der im Folgenden noch beschrieben werden wird. Aus diesem Grund ist die Energiespeichereinheit 4a in Figur 1A schematisch innerhalb des Energieempfangsabschnitts 5 dargestellt.

Figur 1B zeigt einen Längsschnitt des erfindungsgemäßen Energieempfangsabschnitts 5 des Implantates gemäß einer ersten Variante.

Dieser beinhaltet eine Spule 6 mit beispielsweise 1000 Windungen (W = 1000). Besonders bevorzugt liegt W weit unter 1000 und beträgt W < 50, 40, 30, 20, 10.

Die Spule 6 ist auf und um den erwähnten Kern 7 gewickelt, der sich entlang einer Spulenachse SA erstreckt. In dieser Variante ist der Kern 7 eine Vollwelle. Die Spulenachse SA entspricht auch einer Längsachse des Implantates 100 bzw. des Gehäuses 1.

An den jeweiligen Enden der Spule 6 bzw. des Kerns 7 befindet sich bevorzugt ein Feldkollektor 18a und bevorzugt ein weiterer Feldkollektor 18b, die aus über die Spulenenden überstehenden Abschnitten der Vollwelle gebildet sind, um das Feld in der Spule 6 zu homogenisieren. Der Feldkollektor 18a und/oder der weitere Feldkollektor 18b sind bevorzugt so dimensioniert, dass deren Abmessungen quer zur Spulenachse SA identisch sind zu denen des Kerns 7. Insoweit verlängern der/die Feldkollektor(en) 18a, 18b in dieser Variante lediglich den Kern 7 über seine in Richtung der Spulenachse SA liegenden Enden hinaus. Ende des Kerns entspricht dem Ende der Spule 6 in Richtung der Spulenachse SA.

Ein senkrecht zur Spulenachse SA gemessener Durchmesser des Kerns 7 (und der Feldkollektoren 18a, 18b) beträgt in Figur 1B 1mm bis 3 mm(Millimeter). Folglich hat auch die hierauf gewickelte Spule 6 einen entsprechenden Innendurchmesser von 1 mm bis 3 mm.

Eine Länge in Richtung der Spulenachse SA des/der Feldkollektor(en) 18a, 18b beträgt in dieser Alternative bevorzugt mindestens 1%, 2%, 3%, 4%, 5%, 6%, 7%, 8%, 9%, 10%, 20%, 30%, 40%, 50%, 60%, 70%, 80%, 90%, 100% der Gesamtlänge des sich innerhalb der Spule 6 befindlichen Kerns 7. In Figur 1B liegt diese Länge konkret zwischen 83% und 65%, besonders bevorzugt bei 70%, der Gesamtlänge des Kerns 7.

Der Kern 6 und beide Feldkollektoren 18a, 18b haben bevorzugt einen kreisförmigen, senkrecht zur Spulenachse SA verlaufenden Querschnitt. Alternativ kann der Querschnitt auch rechteckig, insbesondere quadratisch sein.

Aus Zusammenschau von Figur 1A und 1B ist verständlich, dass die eine Energiespeichereinheit 4a des Energiespeichers 4 radial zur Spulenachse SA zumindest abschnittsweise um den Kern 7 angeordnet sein kann.

Bevorzugt umfasst die Energiespeichereinheit 4a vollständig den Kern 7, wie es in Figur 2A gezeigt ist.

Der Energiespeicher bzw. die Energiespeichereinheit 4a gemäß Figur 2A besitzt ein, bevorzugt einziges, Gehäuse, das an die Außenkontur bzw. Außenoberfläche der Spule 6 angepasst ist.

Der Kern 7 und die Spule 6 haben einen senkrecht zur Spulenachse SA verlaufenden kreisförmigen Querschnitt. Demzufolge ist die Innenoberfläche bzw. die der Spule 6 zugewandte Oberfläche des Gehäuses des Energiespeichers 4a im Querschnitt (quer zur Spulenachse SA) ringförmig.

In Figur 2A ist der Energiespeicher 4a in einem einzigen, den Kern 7 vollständig umlaufenden Gehäuse aufgenommen. Beispielsweise kann das Gehäuse des Energiespeichers 4 für diese Anordnung um den Kern 7 bzw. die Spule 6 gewickelt oder gebogen werden.

Alternativ kann der Energiespeicher 4a aus einer Vielzahl von Energiespeichereinheiten aufgebaut sein, die jeweils ein Gehäuse aufweisen, das einem Kreissegment um den Kern 7 herum entspricht. Bündig zusammengesetzt umlaufen die Energiespeichereinheiten den Kern 7 dann vollständig oder teilweise.

Figur 2A zeigt im Vergleich zu Figur 1B nicht nur den Energieempfangsabschnitt 5, sondern das gesamte Implantat 100 im Längsschnitt und einer perspektivischen Ansicht.

Durch diese Anordnung des Energiespeichers bzw. der Energiespeichereinheiten 4a erhält das gesamte Implantat einen sehr kompakten Aufbau.

Die bevorzugte, weitere Energiespeichereinheit 4b kann in Richtung der Spulenachse SA neben der Energiespeichereinheit 4a auf der Spule 6 oder neben der Spule 6 auf einem der Feldkollektoren 18a, 18b gemäß Figur 1B angeordnet sein.

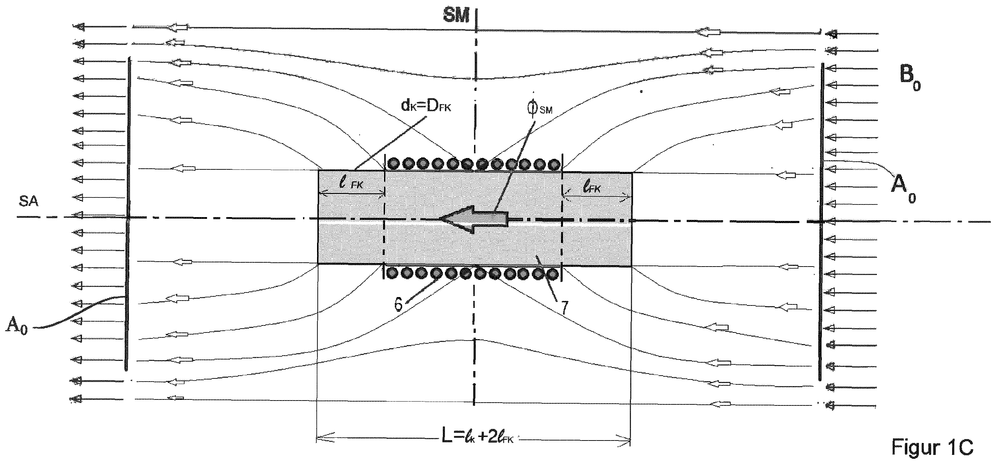

Eine Länge $l_K$ des Kerns 7 mit den Feldkollektoren 18a, 18b gemäß Figur 1B oder 1C kann 15 mm bis 40 mm, insbesondere 15 mm bis 25 mm, betragen, wobei Abmessungen des Feldkollektors 18a und des weiteren Feldkollektors 18b in Richtung der Spulenachse bevorzugt im Bereich $0 < l_K \leq 6{,}0$ mm betragen, weil am äußeren Ende der Feldkollektoren die Gefahr besteht, dass das Nutzfeld negativ wird.

Die Erfindung ist nicht auf die genannten Abmessungen beschränkt. Diese sind lediglich beispielhaft.

Die Feldkollektoren 18a, 18b können gegenüber dem Kern 7 gesonderte Elemente sein oder integrale Bestandteile des Kerns 7 sein.

Figur 1B zeigt beide Feldkollektoren 18a, 18b monolithisch mit dem Kern 7 aus einem einheitlichen magnetisch leitenden Material. Das Material ist beispielsweise ein Ferrit. Der monolithische Aufbau ist besonders bevorzugt für den Fall, dass das Material ein Isolator oder ein zumindest elektrisch schlecht leitendes Material ist, beispielsweise ein Ferrit, weil keine oder kaum Wirbelströme auftreten.

Allgemein ist der Kern und/oder die Feldkollektoren 18a, 18b aus einem Material mit einer hohen relativen magnetischen Permeabilität $\mu_r$ (besonders bevorzugt in einem Bereich von 1000), mit einer möglichst hohen Sättigungsflussdichte (beispielsweise 0,4 bis 0,7 Tesla bei Ferriten; oder 1 bis 1,5 Tesla bei dem weiter unten genannten amorphen Metallen, wie SiFe) und einer möglichst geringen elektrischen Leitfähigkeit, bevorzugt einem Isolator, gebildet.

Die Anordnung der Feldkollektor(en) 18a, 18b ist zwar nur ein bevorzugter aber wesentlicher Grund dafür, dass die Energiespeichereinheiten 4a, 4b des Implantats 100 durch Induktion auch bei Frequenzen von 200 kHz bis 1,5 MHz bei kleiner Baugröße aufgeladen werden können.

Wenn die Energiespeichereinheiten 4a, 4b des Implantats 100 aufgeladen werden müssen, erzeugt ein nicht gezeigtes Ladegerät ein magnetisches Wechselfeld mit einer magnetischen Flussdichte (B-Feld) $B_0$ von ungefähr 0,1 mT bis 1 mT(milli Tesla), die jeweils in einem weiten, das Implantat 100 einschließenden, Bereich homogen vorliegt. Das Feld ist insbesondere bevorzugt in Richtung der Spulenachse SA ausgerichtet (B-Vektor) und durchsetzt die Spule 6.

Das magnetische Wechselfeld ist streng genommen ein elektromagnetisches Wechselfeld. Die elektrische Komponente dieses Feldes ist allerdings von untergeordneter Bedeutung, weshalb im Rahmen dieser Anmeldung lediglich von dem magnetischen Wechselfeld gesprochen wird. Ein reines magnetisches Wechselfeld ist allerdings von der Erfindung mit umfasst.

Die Frequenz f des magnetischen Wechselfeldes liegt in dem oben angegebenen Bereich, beispielsweise bei 500 kHz.

Weil der Energieempfangsabschnitt 5 den erläuterten Kern 7 mit den Feldkollektoren 18a, 18b besitzt, bekommt der Kern 7 hierüber ausreichend Feld, damit die Spule 6 einen ausreichend hohen Lade(wechsel)strom $I_L$ zur Aufladung der Energiespeichereinheiten 4a, 4b erzeugt, den die Ladeelektronik 9 zu dem gleichgerichteten Ladestrom $I_{GL}$ gleichrichtet.

Figuren 1C zeigt magnetische Eigenschaften des Kerns 7 mit Feldkollektoren 18a und 18b.

Figur 1C zeigt schematisch Magnetfeldeinfangflächen $A_0$.

Die Magnetfeldeinfangflächen $A_0$ ergeben sich aus den Dimensionierungen der Feldkollektors 18a, 18b, und des Kerns 7. Die gezeigten Magnetfeldeinfangflächen $A_0$ sind in Bezug auf die gezeigte Konstruktion die entsprechenden Magnetfeldeinfangflächen, bei denen die parallelen Feldlinien des externen magnetischen Wechselfeldes ihre Richtung durch den Energieempfangsabschnitt beginnen zu ändern.

Abmessungen der Magnetfeldeinfangflächen $A_0$ ergeben sich unter Verwendung der Nomenklatur aus Figur 1B in Näherung für Baugrößen des Gehäuses $1\text{cm}^3 \leq V_G \leq 4\text{cm}^3$ und $\mu_r = 10^3$ ($\mu_r$ der Konstruktion aus Kern und Feldkollektoren, und spiegelsymmetrischen Aufbau) aus

$$(I_K + 2I_{FK} + D_{FK})^2 * PI/8 < A_0 < (I_K + 2I_{FK} + D_{FK})^2 * PI/4,$$

und exakt aus $A_0 = \Phi_{SM}/B_0$. Hieraus wird ersichtlich, dass $A_0$ eine fundamentale Größe für die Konstruktion des Implantates bzw. Herzschrittmachers ist.

Die Magnetfeldeinfangflächen $A_0$ befinden sich entlang der Spulenachse SA in einem bestimmten Abstand von dem jeweiligen Feldkollektor 18a, 18b und verlaufen jeweils senkrecht zur Spulenachse SA. Sie sind jeweils wesentlich größer als der entsprechende Feldkollektor 18a, 18b. Maximal ist $A_0 <= 2{,}5*10^{-3}\text{m}^2$.

Das externe magnetische Wechselfeld ($B_0$) ist konstruktionsbedingt durch das Ladegerät nahezu homogen.

Diejenigen magnetischen Feldlinien, die die Magnetfeldeinfangflächen $A_0$ dursetzen, treten über den jeweiligen Feldkollektor und den Kern 7 ein und durchlaufen die in Richtung der Spulenachse SA liegende Längsmitte LM der Spule 6. Verschiebt man die Magnetfeldeinfangfläche $A_0$ durch die Konstruktion in Richtung des jeweiligen Feldkollektors, verkleinert sie sich. Verschiebt man sie hingegen virtuell in die entgegengesetzte Richtung, bleibt sie konstant und je nach $\mu_r$ maximal $A_0$.

Kehrt das Ladegerät die Polarität des magnetischen Wechselfelds um, ist die Situation identisch, mit dem Unterschied, dass die die Magnetfeldeinfangfläche durchlaufenden Feldlinien in den jeweils anderen Feldkollektor und Kern 7 eintreten und an dem gegenüberliegenden Feldkollektor wieder austreten.

Die Magnetfeldeinfangflächen $A_0$ ergeben sich, wie erwähnt, exakt jeweils aus dem maximalen magnetischen Fluss $\Phi_{SM}$ durch die magnetische Längsmitte bzw. eine Querschnittsfläche der Spule an einem bestimmten Ort innerhalb der Spule 6 und der gemittelten externen Flussdichte $B_0$ des externen elektromagnetischen Wechselfeldes über $A_0$ nach der Beziehung $A_0 = \dfrac{\Phi_{SM}}{B_0}$. Im Falle des gezeigten spiegelsymmetrischen Aufbaus des Energieempfangsabschnittes ist dieser Ort die Längsmitte SM der Spule.

Im Vorhergehenden ist zur Erläuterung der Aufladung des Energiespeichers ein magnetisches Wechselfeld mit < 1mT und mit einer Frequenz von 500kHz angenommen worden.

Die Erfindung ist hierauf nicht beschränkt. Die Erwägungen vor der Figurenbeschreibung hinsichtlich des Ladestromes $I_{GL}$, der Windungszahl W, dem magnetischen Fluss $\Phi_{SM}$ und der Frequenz des magnetischen Wechselfeldes, dem ohmschen Widerstand der Spule und einem optionalen Kompensationskondensator gelten für die Ausführungsform gleichermaßen.

Aufgrund der Abmessungen des Feldkollektors 18a und des weiteren Feldkollektors 18b liegt eine verstärkte Kernflussdichte $B_K$ innerhalb des Kerns 7 vor. Die Kernflussdichte $B_K$ übersteigt die magnetische Flussdichte $B_0$ zum Beispiel bis zu dem 200-fachen ($B_K = 200B_0$).

Wenn die magnetische Flussdichte $B_0$ des von dem Ladegerät erzeugten magnetischen Wechselfeldes, die im Bereich des Implantats vorliegt, 0,1 mT beträgt, beläuft sich folglich die Kernflussdichte $B_K$ im unbelasteten Zustand auf ca. 20mT. Vermindert wird die genannte Kernflussdichte $B_K$ allerdings um das auftretende Gegenfeld innerhalb der Spule 6, das von dem Lade(wechsel)strom $I_L$ herrührt. Der Ladestrom liegt bei 400 mA.

Die genannten Werte erlauben die Energiespeichereinheiten 4a, 4b, die zusammen bevorzugt einen Ladungsinhalt von 400 As besitzen, in ca. 15 min bis 20 min aufzuladen.

Die Abmessungen des Kerns 7 bzw. der Feldkollektoren 18a, 18b, die Parameter der Spule 6 und die restlichen Elemente werden bevorzugt so gewählt, dass das Gewicht des gesamten Implantates 100 gering ist und im Bereich von 4g (Gramm), bevorzugt unter 3g, liegt.

Der Ladestrom wird von der Spule 6 dem Energiespeicher bzw. den Energiespeichereinheiten 4a, 4b bevorzugt über die gezeigte Ladeelektronik 9 zugeführt.

Besonders bevorzugt kann als zu Ferrit alternatives Material ein amorphes Metall, beispielsweise SiFe, für den Kern 7 und/oder die Feldkollektoren 18a, 18b verwendet werden. Erhältlich auf dem Markt ist ein solches Metall zum Beispiel unter dem Markennamen ARNON.

Der Kern 7 und/oder die Feldkollektoren 18a, 18b können bevorzugt einen Schichtaufbau besitzen mit einzelnen Schichten aus den genannten Materialien (bspw. Ferrit oder SiFe) und sind dann bevorzugt nicht mehr kreisförmig sondern quadratisch.

Die Elektronik 3 und die Ladeelektronik ist in der Variante gemäß Figur 2A auf einer mit einem Durchgang versehenen Platine oder einer biegsamen Platine angeordnet. Die Platine wird wie gezeigt auf die Vollwelle geschoben bzw. um die Vollwelle gebogen und letztendlich mit den Anschlüssen 41a des Energiespeichers bzw. der Energiespeichereinheit 4a kontaktiert.

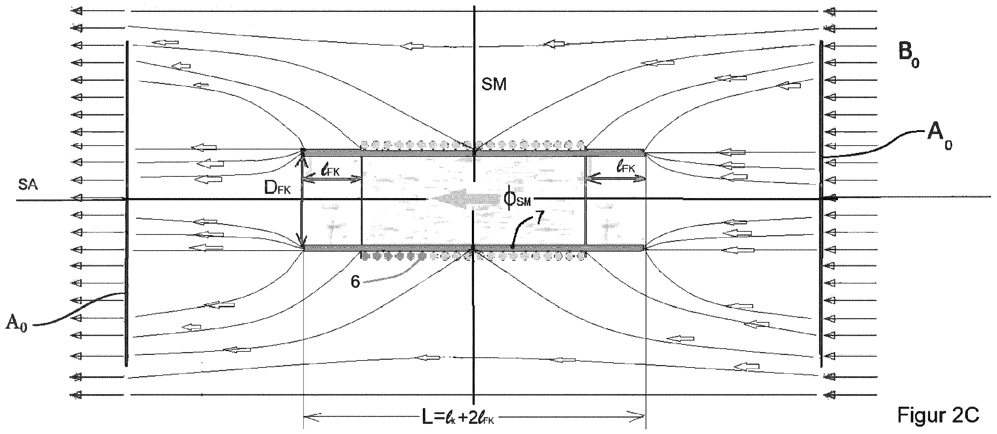

Figur 2B zeigt eine weitere Variante des erfindungsgemäßen Implantates 100, die sich von der aus Figur 2A gezeigten zum einen dadurch unterscheidet, dass Kern 7a und Feldkollektoren 28a, 28b als durchgehende Hohlwelle ausgebildet sind. Figur 2C zeigt den Kern 7a und die Hohlwelle ohne Gehäuse sowie die entsprechenden magnetischen Eigenschaften des Kerns 7a mit Feldkollektoren 28a und 28b. Die Ausführungen zu Figur 1C gelten analog für Figur 2C.

Zum anderen sind der Energiespeicher 4 bzw. die Energiespeichereinheit 4a und die Platine mit der Elektronik 3 und der Ladeelektronik in die Hohlwelle eingeführt. Die Elektronik 3 wird bevorzugt 3-dimensional verdrahtet und eingegossen als Körper in die Hohlwelle eingesetzt.

Bevorzugt ist die Hohlwelle 7a aus dem magnetisch leitenden Material gebildet, das in Bezug auf Figuren 1A, 1B, 2A bereits genannt sind. Die Spule 6 ist auf eine Außenoberfläche der Hohlwelle gewickelt. Besonders bevorzugt sind zwei Energiespeichereinheiten innerhalb der Hohlwelle 7a aufgenommen, zwischen denen sich die Elektronik 3 befindet und die jeweils am äußeren Ende innerhalb der Hohlwelle 7a sitzen, um den magnetische Fluss in der Spule 6 zu homogenisieren und zu erhöhen und die Felddichte auf der Oberfläche der magnetisch leitenden Hohlwelle 7a zu verringern.

Alternativ kann die Hohlwelle lediglich einen Trägerkörper für die Spule 6 bilden, der aus einem magnetisch nichtleitenden Material gebildet ist. In diesem Fall ist die Spule 6 eine Luftspule.

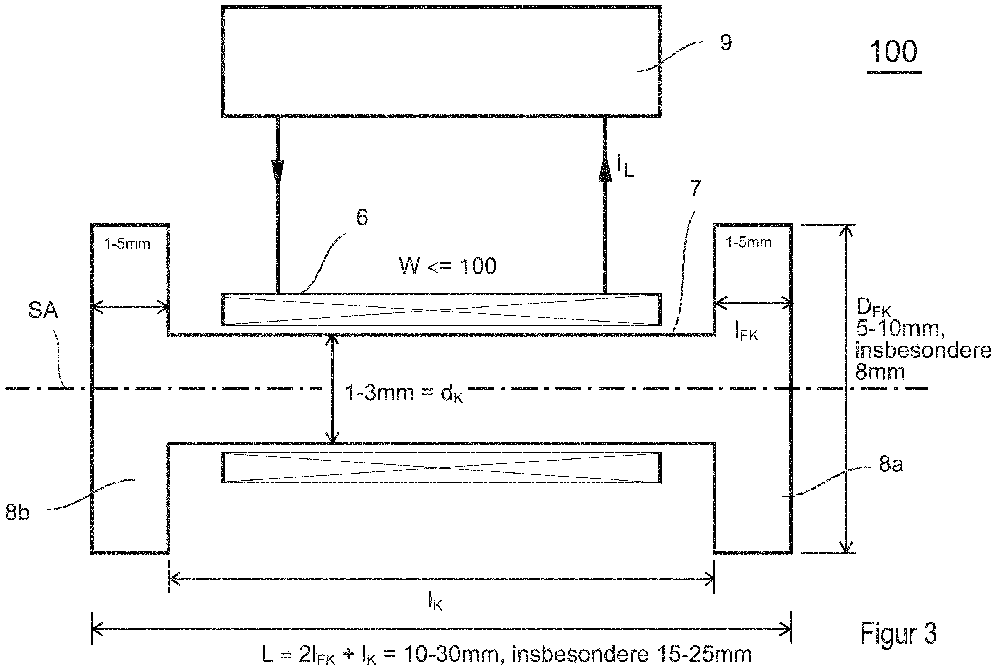

Figur 3 zeigt einen Längsschnitt eines alternativen erfindungsgemäßen Energieempfangsabschnitts 5 des Implantates 100, wobei sich der gezeigte Energieempfangsabschnitt 5 von dem aus Figur 1B lediglich dadurch unterscheidet, dass Feldkollektoren 18a, 18b vorgesehen/ausgebildet sind, die eine größere Querschnittsfläche als die des Kerns 7 aufweisen. Der Durchmesser $D_{FK}$ der Feldkollektoren 18a, 18b beträgt zwischen 5mm und 10mm.

Der in Figur 3 gezeigte Kern 7 und bevorzugt die Feldkollektoren 18a, 18b können auch als Hohlwelle ausgebildet sein.

Alle übrigen Elemente des Energieempfangsabschnitts 5 aus Figur 3 sind mit denen aus Figuren 1A und 1B identisch, weshalb auf die dortigen Ausführungen verwiesen wird. Auch kann der Energieempfangsabschnitt 5 in das in Figuren 2A und 2B gezeigte Gehäuse 1 mit allen anderen Komponenten eingesetzt werden. Insbesondere die Anordnung der Energiespeicher 4 bzw. Energiespeichereinheiten 4a kann zu Figuren 2A und 2B identisch sein. Die Elektronik 3, 9 kann an einem der Feldkollektoren 8a, 8b anliegen, in einer darin gebildeten Ausnehmung aufgenommen und/oder wie in Figur 3B gezeigt im Kern 7 angeordnet sein.

Die Magnetfeldeinfangfläche $A_0$ hat gleichermaßen die bereits genannte Größe $A_0 <= 2,5*10^{-3}m^2$.

Folgende Ausführungen gelten für alle in den Figuren 1 bis 3 gezeigten Varianten des Implantates.

Die Elektronik 3 ist bevorzugt eingerichtet, Informationen zur räumlichen Anpassung/Korrektur des Vektors (B-Vektor) des magnetischen Wechselfeldes der Ladespule des Ladegerätes an die Spulenachse an das Ladegerät zu liefern, wodurch das Implantat 100 in einer beliebigen räumlichen Orientierung implantierbar wird.

Beispielsweise ist die mit dem Elektrodenabschnitt 2 verbundene Elektronik 3 eingerichtet, die Informationen zu generieren zur bevorzugt automatischen - vorzugsweise parallelen - Ausrichtung der Spulenachse des Ladegerätes zur Spulenachse des Implantates. Die Informationen sind insbesondere Zeitinformationen in einer Zeiteinheit, wie beispielsweise Sekunden.

*Erfindungsgemäß ist die Elektronik (3) eingerichtet, (i) ein Startsignal zum Starten der Anpassung der Ausrichtung des magnetischen Wechselfeldes an das Ladegerät über eine nicht gezeigte Kommunikationseinheit zu senden oder über die Kommunikationseinheit von diesem zu empfangen, wobei das Ladegeräte daraufhin die Ausrichtung des magnetischen Wechselfelds gemäß einer Bewegungsfunktion verändert, und*

*(ii) anschließend über die Kommunikationseinheit die Zeitinformationen, die angeben, wann der Ladestrom zu der Wiederaufladung geeignet war an das Ladegerät als die Informationen zur Ausrichtung der Spulenachse des Ladegerätes auszugeben.*

Die Kommunikationseinheit versendet die Zeitinformationen an die Außenwelt, wobei eine übergeordnete, die Zeitinformationen empfangende Einheit, wie beispielsweise das Ladegerät (bevorzugt gemäß EP 4035728 A1), aus den Zeitinformationen auf die beliebige Lage und Ausrichtung des Implantates schließen kann. Mit Kenntnis der Lage und Ausrichtung des Implantates kann die übergeordnete Einheit, wie bspw. das Ladegeräte den B-Feldvektor des magnetischen Wechselfeldes zur Optimierung der Aufladung entsprechend ausrichten.

Hervorzuheben ist hierbei, dass die anfängliche Ausrichtung des B-Feldvektors beliebige Richtungen im Raum einnehmen kann, weil immer eine anschließende Anpassung an die Lage und Ausrichtung des Implantates möglich ist. Auch folgt hieraus, dass bei Implantation des Implantates keine Beachtung der resultierenden Lage und Ausrichtung des Implantates geschenkt werden muss.

Die Bewegungsfunktion, gemäß der das Ladegerät die Ausrichtung des magnetischen Wechselfeldes, bspw. den B-Feldvektor, ändert, kann beliebig sein und bedarf auch keiner besonderen Startausrichtung des Feldes.

Erforderlich ist lediglich, dass die Bewegungsfunktion eine Funktion in Abhängigkeit von der Zeit ist (f(t)), aus der das Ladegerät nach Durchlaufen der Bewegungsfunktion schließen kann, zu welchem Zeitpunkt das magnetische Wechselfeld welche Ausrichtung hatte, wenn das Ladegerät zeitlich mit dem Implantat synchronisiert war.

Empfängt das Ladegerät nach Durchlaufen der Bewegungsfunktion nunmehr die Zeitinformationen von dem erfindungsgemäßen Implantat, kann es aus der Bewegungsfunktion und den Zeitinformationen die entsprechende Ausrichtung ermitteln.

Das Ladegerät kann die im Vorhergehenden angesprochene Ausrichtung des Wechselfeldes gemäß folgenden Optionen vornehmen:

- Das Ladegerät kann eine Körperhalterung (bspw. Stuhl oder Liege), auf der sich der Körper befindet, bevorzugt um zwei orthogonale Achsen drehen und/oder linear versetzen; und/oder
- Das Ladegerät kann eine Ladespule, die das magnetische Wechselfeld erzeugt, bevorzugt um zwei orthogonale Achsen drehen und/oder linear versetzen; und/oder
- Das Ladegerät kann das magnetische Wechselfeld, das bevorzugt durch eine Vielzahl von Ladespulen erzeugt werden, dadurch ausrichten, dass Betriebsparameter der Ladespulen verändert werden und sich individuelle magnetische Wechselfelder der einzelnen Ladespulen zu dem magnetischen Wechselfeld mit einer bestimmten Ausrichtung überlagern.

*Besonders bevorzugt ist (V) die Elektronik eingerichtet ist,*

*(i) einen Abschluss der räumlichen Anpassung der Ausrichtung des magnetischen Wechselfeldes zu erkennen oder von dem Ladegerät über die Kommunikationseinheit signalisiert zu bekommen, und*
*(ii) anschließend die Zeitinformationen auszugeben.*

*Bevorzugt geben die Zeitinformationen zumindest einen Zeitpunkt oder einen Zeitbereich an, zu dem/in dem der Ladestrom zur Wiederaufladung maximal war.*

Der Zeitpunkt ist insbesondere ein solcher Zeitpunkt, an dem der Ladestrom ein Maximum erreichte. Der Zeitbereich hingegen ist eine Zeitspanne, in der der Ladestrom ein Maximum durchließ.

Insbesondere ist der Zeitbereich dadurch definiert, dass er den Zeitpunkt des maximalen Ladestroms und zeitlich davor und danach liegende Zeitpunkte, in denen der Ladestrom maximal x% unter dem Maximum lag, beinhaltet, wobei x%=1%, 2%, 3%, 4%, 5%, 6%, 7%, 8%, 9%, 10% ist.

*Insbesondere bevorzugt geben die Zeitinformationen in Form von Zeitpunkten oder Zeitbereichen an, an denen/in denen der Ladestrom maximal war, wobei die Elektronik eingerichtet ist, (i) die Zeitpunkte oder Zeitbereiche dahingehend zu qualifizieren, ob der jeweilige maximale Ladestrom ein globales oder lokales Maximum darstellt, und (ii) zumindest den Zeitpunkt oder den Zeitbereich gemäß dem globalen Maximum, beispielsweise an das Ladegerät, als die Informationen zur Anpassung des magnetischen Wechselfeldes auszugeben.*

Besonders bevorzugt übermittelt das Implantat 100 Zeitpunkte/Zeitbereiche verschiedener Maxima, insbesondere aller Maxima, an das Ladegerät. Das Ladegerät kann anschließend entscheiden, welche Ausrichtung des magnetischen Wechselfeldes es für die Wiederaufladung einstellt.

Bevorzugt ist das erfindungsgemäße Implantat 100 ausgestaltet, dass die Elektronik 3 die Stärke des Ladestroms in regelmäßigen oder unregelmäßigen Intervallen bestimmt und hierauf basierend die Zeitinformationen ermittelt und für das Ausgeben abspeichert.

Bevorzugt speichert die Elektronik die Stärken des Ladestroms nicht über den gesamten Zeitraum vom Startsignal für die Anpassung bis Abschluss der Anpassung, sondern nur deren Maxima zur späteren Ausgabe mit der entsprechenden Zeitinformation.

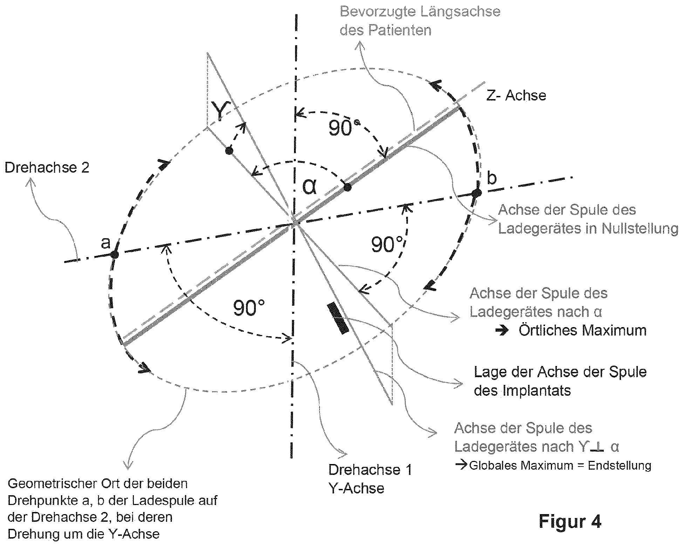

Figur 4 dient der Erläuterung des entsprechenden Korrekturverfahrens und zeigt das Implantat 100 in beliebiger Position im Körper eines Menschen bzw. Patienten.

Das Ladegerät ist insbesondere gemäß EP 4035728 A1 aufgebaut, wobei der Aufbau des Ladegerätes hier mit einbezogen wird und die im Folgenden genannten Drehachsen der Ladespule sich auf die in EP 4035728 A1 gezeigten Drehachsen beziehen.

Die räumliche Ausrichtung wird insbesondere durch Rotationsbewegungen der Ladespule erreicht, wobei durch bevorzugt nur zwei Rotationsschritte die deckungsgleiche räumliche Lage mit der Achse der Spule des Ladegerätes erzwungen wird als Ausgangslage für eine optimale Aufladung des Energiespeichers/Akkumulators des Implantats 100.

Diese Rotationsbewegungen/-schritte werden nachfolgend erläutert: Der Patient liegt mit dem Implantat 100 im inneren der Spule des Ladegerätes z.B. in Richtung deren Spulenachse (z-Achse).

Im ersten Schritt wird die Spule des Ladegerätes - ausgehend von der gezeigten Nullstellung - um eine zur Spulenachse orthogonalen Achse (Drehachse 1/Y-Achse) um bspw. +/-70° oder bei entsprechenden räumlichen ausreichenden Abmessungen der Ladespule um maximal 180° gedreht. Dabei überfährt die Spulenachse des Ladegerätes bei dem Winkel Alpha ein lokales (im Sonderfall globales) Maximum, festgestellt durch eine im Implantat aufgetretene maximale Amplitude des Stromes, wobei das Implantat die entsprechende Zeitinformation ausgibt.

Im nächsten Schritt wird die Achse (Drehachse 1/Y-Achse) der Ladespule auf den Winkel Alpha zurückgedreht und um die zweite, zur Spulenachse (Drehachse 2) orthogonalen Achse gedreht. Dabei wird beim Winkel Gamma ein zweites Maximum überfahren, festgestellt wieder mit Hilfe der maximalen Stromamplitude der Spule des Implantats, wobei das Implantat die entsprechende Zeitinformation wieder ausgibt.

Das bei dem Winkel Gamma gefundene Maximum bildet immer das globale Maximum und ist zugleich die optimale Ausrichtung des Ladegerätes bei der die Richtung der Achse der Spule des Implantates und die der Spule des Ladegerätes zusammenfallen.. Wurde im Sonderfall im ersten Schritt kein Strommaximum festgestellt, dann liegt die Achse der Spule des Implantats in Richtung der Drehachse 1/Y-Achse (einfachster Fall), weshalb die Spulenachse in Richtung der Drehachse 1/Y-Achse gebracht werden muss und das dort gefundene Maximum ein globales Maximum darstellt. Das Ladegerät leitet den Umstand, dass kein Maximum festgestellt wurde, bevorzugt daraus ab, dass es in einem bestimmten Zeitraum keine Zeitinformationen vom Implantat erhält und dreht seine Achse um 90°.

Durch diese Ausgestaltung kann ein weitgehend optimaler Ladestrom bei jeder Position des Implantates im Körper erzielt werden.

Die Ausführungen vor der Figurenbeschreibung gelten für die Ausführungsform und die erläuterten Ausgestaltungen und Modifikationen entsprechend und vice versa.

**Patentansprüche**

1.  Elektronisches Implantat (100) zur Implantation in einen Körper eines Lebewesens und zur Überwachung einer Körperfunktion, insbesondere Schrittmacher zur Überwachung und Steuerung der Körperfunktion, wobei das Implantat (100) aufweist:

    einen Elektrodenabschnitt (2), der bestimmungsgemäß an einem Körperabschnitt zu befestigen oder anzuordnen ist; und
    ein Gehäuse, das ein Volumen $V_G$ im Bereich von $0,5 \leq V_G \leq 4\ cm^3$, bevorzugt $\leq 2 cm^3$, umfasst und das folgende Komponenten des elektronischen Implantates (100) aufnimmt:

    (i) eine mit dem Elektrodenabschnitt verbundene Elektronik (3), die eingerichtet ist, zumindest die Körperfunktion über den Elektrodenabschnitt (2) zu überwachen;
    (ii) einen Energiespeicher (4) zur Langzeitversorgung der Elektronik (3) mit elektrischer Energie, der nach Entladung mit elektrischer Energie wieder aufgeladen werden kann; und
    (iii) einen mit dem Energiespeicher (4) elektrisch verbundenen Energieempfangsabschnitt (5), der derart eingerichtet ist, dass er Energie kontaktlos empfangen und an den Energiespeicher (4) zum Wiederaufladen des Energiespeichers (4) abgeben kann; wobei

    (I) der Energieempfangsabschnitt (5) zumindest eine Spule (6), die sich entlang einer Spulenachse (SA) erstreckt und eingerichtet ist, die Energie zu empfangen und an den Energiespeicher (4) abzugeben, wenn sie von einem magnetischen Wechselfeld, das ein externes Ladegerät erzeugt, durchsetzt wird, wobei die Spule eine Luftspule ist oder einen magnetisch leitenden Kern (7), der sich in der Spule (6) befindet und entlang der Spulenachse (SA) verläuft, beinhaltet, wobei

    a) der Kern (7) entlang der Spulenachse (SA) verläuft und über Enden der Spule (6) hinaus nicht vorsteht, oder
    b) der Kern (7) entlang der Spulenachse (SA) verläuft und zur Ausbildung eines Feldkollektors (18a, 18b) über mindestens ein Ende der Spule (6) hinaus vorsteht, ohne dass sich eine Querschnittsfläche des Kerns (7) vergrößert, oder
    c) der Kern (7) entlang der Spulenachse (SA) verläuft und zur Ausbildung eines Feldkollektors (18a, 18b) über mindestens ein Ende der Spule (6) hinaus vorsteht, wobei sich eine Querschnittsfläche des Kerns (7) vergrößert, und

(II) die Spule (6), wenn sie von dem magnetischen Wechselfeld durchsetzt wird, einen durch einen Gleichrichter gleichgerichteten Ladestrom von bevorzugt maximal 2A erzeugt, der dem Energiespeicher (4) zum Wiederaufladen zugeführt wird;

(III) der Energieempfangsabschnitt (5) eine senkrecht zur Spulenachse (SA) stehende Magnetfeldeinfangfläche $A_0$ mit $A_0 <= 2{,}5*10^{-3}m^2$ besitzt, die durch $A_0 = \Phi_{SM}/B_0$ definiert ist, wobei $\Phi_{SM}$ der magnetische Fluss ist, der eine in Richtung der Spulenachse (SA) liegende magnetische Längsmitte innerhalb der Spule (6) als Maximum durchsetzt, und $B_0$ die externe, mittlere Flussdichte des magnetischen Wechselfeldes über der Magnetfeldeinfangfläche $A_0$ ist; und

(IV) die Elektronik (3) eingerichtet ist, Informationen zur automatischen Korrektur einer Abweichung zwischen einer räumlichen Ausrichtung eines Vektors eines inneren magnetischen Wechselfeldes einer Ladespule eines Ladegerätes und der Spulenachse des Implantates zum Beispiel an das Ladegerät zu liefern, wodurch das Implantat (100) in einer beliebigen räumlichen Orientierung implantierbar ist, wobei

gemäß (IV) die Elektronik (3) eingerichtet ist, (i) ein Startsignal zum Starten der Anpassung der Ausrichtung des magnetischen Wechselfeldes an das Ladegerät zu senden oder von diesem zu empfangen, das daraufhin die Ausrichtung des magnetischen Wechselfelds verändert, und (ii) Zeitinformationen, die angeben, wann der Ladestrom zu der Wiederaufladung geeignet war, beispielsweise an das Ladegerät, als die Informationen zur Korrektur auszugeben.

2. Implantat (100) nach Patentanspruch 1, wobei
die Zeitinformationen zumindest einen Zeitpunkt oder einen Zeitbereich angeben, zu dem die Amplitude des Ladestromes oder sein Gradient zur Aufladung maximal war..

3. Implantat (100) nach Patentanspruch 2, wobei

die Zeitinformationen Zeitpunkte oder Zeitbereiche angeben, an denen/in denen der Ladestrom maximal war, und
die Elektronik (3) eingerichtet ist, (i) die Zeitpunkte oder Zeitbereiche dahingehend zu qualifizieren, ob der jeweilige maximale Ladestrom ein globales oder lokales Maximum darstellt, und (ii) zumindest den Zeitpunkt oder den Zeitbereich gemäß dem globalen oder lokalen Maximum beispielsweise an das Ladegerät als die Informationen zur Korrektur auszugeben.

4. Implantat (100) nach einem der vorstehenden Patentansprüche, wobei
die Elektronik (3) die Stärke des Ladestroms in regelmäßigen oder unregelmäßigen Intervallen bestimmt und hierauf basierend die Zeitinformationen ermittelt und für das Ausgeben abspeichert.

5. Implantat (100) nach Patentanspruch 1, 2, 3 oder 4, wobei
(V) die Elektronik (3) eingerichtet ist, (i) einen Abschluss der räumlichen Anpassung der Ausrichtung des magnetischen Wechselfeldes zu erkennen oder von dem Ladegerät signalisiert zu bekommen, und (ii) anschließend die Zeitinformationen an das Ladegerät auszugeben.

6. Elektronisches Implantat (100) zur Implantation in einen Körper eines Lebewesens und zur Überwachung einer Körperfunktion, insbesondere Schrittmacher zur Überwachung und Steuerung der Körperfunktion, wobei das Implantat (100) aufweist:

einen Elektrodenabschnitt (2), der bestimmungsgemäß an einem Körperabschnitt zu befestigen oder anzuordnen ist; und
ein Gehäuse, das ein Volumen $V_G$ im Bereich von $0{,}5 \leq V_G \leq 4\,cm^3$, bevorzugt $\leq 2cm^3$, umfasst und das folgende Komponenten des elektronischen Implantates (100) aufnimmt:

(i) eine mit dem Elektrodenabschnitt verbundene Elektronik (3), die eingerichtet ist, zumindest die Körperfunktion über den Elektrodenabschnitt (2) zu überwachen;
(ii) einen Energiespeicher (4) zur Langzeitversorgung der Elektronik (3) mit elektrischer Energie, der nach Entladung mit elektrischer Energie wieder aufgeladen werden kann; und
(iii) einen mit dem Energiespeicher (4) elektrisch verbundenen Energieempfangsabschnitt (5), der derart eingerichtet ist, dass er Energie kontaktlos empfangen und an den Energiespeicher (4) zum Wiederaufladen des Energiespeichers (4) abgeben kann; wobei

(I) der Energieempfangsabschnitt (5) zumindest eine Spule (6), die sich entlang einer Spulenachse (SA) erstreckt und eingerichtet ist, die Energie zu empfangen und an den Energiespeicher (4) abzugeben, wenn sie von einem magnetischen Wechselfeld, das ein externes Ladegerät erzeugt, durchsetzt wird, wobei die Spule eine Luftspule ist oder einen magnetisch leitenden Kern (7), der sich in der Spule (6) befindet und entlang der Spulenachse (SA) verläuft, beinhaltet, wobei

a) der Kern (7) entlang der Spulenachse (SA) verläuft und über Enden der Spule (6) hinaus nicht vorsteht, oder

b) der Kern (7) entlang der Spulenachse (SA) verläuft und zur Ausbildung eines Feldkollektors (18a, 18b) über mindestens ein Ende der Spule (6) hinaus vorsteht, ohne dass sich eine Querschnittsfläche des Kerns (7) vergrößert, oder

c) der Kern (7) entlang der Spulenachse (SA) verläuft und zur Ausbildung eines Feldkollektors (18a, 18b) über mindestens ein Ende der Spule (6) hinaus vorsteht, wobei sich eine Querschnittsfläche des Kerns (7) vergrößert, und

(II) die Spule (6), wenn sie von dem magnetischen Wechselfeld durchsetzt wird, einen durch einen Gleichrichter gleichgerichteten Ladestrom von bevorzugt maximal 2A erzeugt, der dem Energiespeicher (4) zum Wiederaufladen zugeführt wird;

(III) der Energieempfangsabschnitt (5) eine senkrecht zur Spulenachse (SA) stehende Magnetfeldeinfangfläche $A_0$ mit $A_0 <= 2,5*10^{-3}m^2$ besitzt, die durch $A_0 = \Phi_{SM}/B_0$ definiert ist, wobei $\Phi_{SM}$ der magnetische Fluss ist, der eine in Richtung der Spulenachse (SA) liegende magnetische Längsmitte innerhalb der Spule (6) als Maximum durchsetzt, und $B_0$ die externe, mittlere Flussdichte des magnetischen Wechselfeldes über der Magnetfeldeinfangfläche $A_0$ ist; und

(IV) die Elektronik (3) eingerichtet ist, Informationen zur automatischen Korrektur einer Abweichung zwischen einer räumlichen Ausrichtung eines Vektors eines inneren magnetischen Wechselfeldes einer Ladespule des Ladegerätes und der Spulenachse des Implantates zum Beispiel an das Ladegerät zu liefern, wodurch das Implantat (100) in einer beliebigen räumlichen Orientierung implantierbar ist, wobei

die Elektronik (3) einen Speicher besitzt, in dem mindestens ein Schwellenwert, der z.B. einer bestimmten Stärke des Ladestromes entspricht, gespeichert ist, und

die Elektronik (3) eingerichtet ist, den Ladestrom mit dem Schwellenwert zu vergleichen, und, wenn der Ladestrom den Schwellenwert erreicht, zumindest diese Information zur automatischen Korrektur der räumlichen Ausrichtung der Spulenachse des Implantates an das Ladegerät unmittelbar und unverzögert zu liefern.

7. Implantat (100) nach einem der vorangehenden Patentansprüche 1 bis 6, wobei der Kern (7) ein magnetisch leitendes Gehäuse des Energiespeichers ist, auf den die Spule (6) gewickelt ist.

8. Implantat (100) nach einem der vorangehenden Patentansprüche 1 bis 6, wobei der Kern (7) eine magnetisch leitende Vollwelle oder eine magnetisch leitende Hohlwelle ist.

9. Implantat (100) nach Patentanspruch 8, wobei der Kern (7) die magnetisch leitende Vollwelle ist und sich der magnetisch leitende Energiespeicher in Richtung der Spulenachse neben der Vollwelle und innerhalb oder außerhalb der Spule befindet.

10. Implantat (100) nach Patentanspruch 8, wobei der Kern (7) die magnetisch leitende Vollwelle ist, auf die die Spule gewickelt ist, und sich der magnetisch nichtleitende Energiespeicher radial zur Spulenachse um die Spule erstreckt.

11. Implantat (100) nach Patentanspruch 8, wobei der Kern (7) die magnetisch leitende Hohlwelle ist, auf die die Spule gewickelt ist, und sich der/die Energiespeicher innerhalb der Hohlwelle befindet/befinden bevorzugt jeweils einer der Energiespeicher am Ende der Hohlwelle.

12. Implantat (100) nach Patentanspruch 8, wobei der Kern (7) die magnetisch leitende Hohlwelle ist, auf die die Spule gewickelt ist, und sich der magnetisch nichtleitende Energiespeicher in Richtung der Spulenachse neben der Hohlwelle und innerhalb oder außerhalb der Spule befindet.

13. Implantat (100) nach einem der vorangehenden Patentansprüche 1 bis 12, wobei die Spule W Wicklungen besitzt und $W \leq 1000$, bevorzugt $W \leq 50, 40, 30, 20, 10$ ist.

**14.** Implantat (100) nach einem der vorangehenden Patentansprüche 1 bis 13, wobei das Implantat den Ladestrom bestimmungsgemäß bei einer Frequenz f des externen magnetischen Wechselfeldes erzeugt, wobei f $\leq$ 2 MHz, vorzugsweise f $\leq$ 500 kHz, 400 kHz, 300 kHz, 200 kHz, 100 kHz, 50 kHz ist.

**15.** Implantat (100) nach einem der vorangehenden Patentansprüche 1 bis 14, wobei der Energieempfangsabschnitt so aufgebaut ist, dass die magnetische Längsmitte mit der Längsmitte der Spule zusammenfällt.

**16.** Implantat (100) nach einem der Patentansprüche 1 bis 14, wobei
(V) die Elektronik (3) eingerichtet ist,

(i) einen Abschluss der räumlichen Anpassung der Ausrichtung des magnetischen Wechselfeldes zu erkennen oder von dem Ladegerät signalisiert zu bekommen, und
(ii) anschließend auf Basis des momentanen Ladestroms dem Ladegerät ein Feldänderungssignal zu senden, das dem Ladegerät signalisiert, Frequenz und/oder Amplitude des magnetischen Wechselfeldes zu erhöhen oder zu vermindern, wobei die Elektronik (3) dem Ladegerät signalisiert, welche Auswirkungen die Änderungen der Frequenz und/oder Amplitude des magnetischen Wechselfeldes nach sich ziehen zur Kompensation der körpereigenen Dämpfung des magnetischen Wechselfeldes.

**Claims**

**1.** Electronic implant (100) for implantation into a body of a living being and for monitoring a bodily function, in particular a pacemaker for monitoring and controlling the bodily function, the implant (100) comprising:

an electrode portion (2) that is, according to its intended purpose, to be attached to or to be arranged at a body portion; and
a housing which encases a volume $V_G$ in the range of $0.5 \leq V_G \leq 4$ cm$^3$, preferably $\leq 2$ cm$^3$, and which accommodates the following components of the electronic implant (100):

(i) an electronics assembly (3) connected to the electrode portion, which is configured to monitor at least the body function via the electrode portion (2);
(ii) an energy storage (4) to long-term-supply the electronics assembly (3) with electrical energy which can be recharged with electrical energy after discharge; and
(iii) an energy receiving portion (5) electrically connected to the energy storage (4), which is configured so as to be able to receive energy without contact and to deliver the energy to the energy storage (4) for recharging the energy storage (4); wherein

(I) the energy receiving portion (5) comprises at least one coil (6) extending along a coil axis (SA) and adapted to receive and deliver the energy to the energy storage (4) when passed through by an alternating magnetic field generated by an external charging device, the coil being an air-core coil or comprising a magnetically conductive core (7) located in the coil and extending along the coil axis, wherein

a) the core runs (7) along the coil axis and does not protrude beyond the ends of the coil (6), or
b) the core (7) extends along the coil axis (SA) and protrudes beyond at least one end of the coil (6) to form a field collector (18a, 18b) without increasing a cross-sectional area of the core (7), or
c) the core (7) extends along the coil axis (SA) and protrudes beyond at least one end of the coil to form a field collector (18a, 18b), wherein a cross-sectional area of the core (7) increases, and

(II) the coil (6), when penetrated by the alternating magnetic field, generates a charging current of preferable 2A at maximum rectified by a rectifier, which is fed to the energy store (4) for recharging;
(III) the energy receiving portion has a magnetic field collecting area $A_0$ perpendicular to the coil axis with $A_0 \leq 2.5*10^{-3}$m$^2$ defined by $A_0 = \Phi_{SM}/B_0$, wherein $\Phi_{SM}$ is the magnetic flux passing, as a maximum, through a longitudinal magnetic center within the coil in the direction of the coil axis and $B_0$ is the external mean flux density of the alternating magnetic field over the magnetic field collecting area $A_0$; and
(IV) the electronics assembly (3) is configured to provide information for automatic correction of a deviation between a spatial orientation of a vector of an internal alternating magnetic field of a charging coil of a charging device and the coil axis of the implant, for example to the charging device, thereby allowing the implant (100) to be implantable in any spatial orientation, wherein

according to (IV), the electronics assembly (3) is configured to (i) send to, or receive from, the charging device a start signal for starting an adjustment of an orientation of the alternating magnetic field, upon which the charging device changes the orientation of the alternating magnetic field, and (ii) output time information indicating when a charging current was suitable for recharging, for example to the charging device, as the information for correction.

2. Implant (100) according to patent claim 1, wherein
the time information indicates at least one point in time or a time range at which the amplitude of the charging current or its gradient for charging was maximum.

3. Implant (100) according to patent claim 2, wherein

the time information indicates times or time ranges at which the charging current was maximum, and
the electronics assembly (3) is configured to (i) qualify the time instants or time ranges as to whether the respective maximum charging current represents a global maximum or a local maximum, and (ii) output at least the time instant or the time range corresponding to the global or local maximum, for example to the charging device, as information for correction.

4. Implant (100) according to any one of the preceding patent claims, wherein
the electronics assembly (3) determines the strength of the charging current at regular or irregular intervals and, based on this, determines the time information and stores it for output.

5. Implant (100) according to patent claim 1, 2, 3 or 4, wherein
(V) the electronics assembly (3) is set up (i) to detect a completion of the spatial adjustment of the alignment of the alternating magnetic field or to be signaled by the charging device, and (ii) subsequently to output the time information to the charging device.

6. Electronic implant (100) for implantation into a body of a living being and for monitoring a bodily function, in particular a pacemaker for monitoring and controlling the bodily function, the implant (100) comprising:

an electrode portion (2) that is, according to its intended purpose, to be attached to or to be arranged at a body portion; and
a housing which encases a volume $V_G$ in the range of $0.5 \leq V_G \leq 4$ cm$^3$, preferably $\leq 2$cm$^3$, and which accommodates the following components of the electronic implant (100):

(i) an electronics assembly (3) connected to the electrode portion, which is configured to monitor at least the body function via the electrode portion (2);
(ii) an energy storage (4) to long-term-supply the electronics assembly (3) with electrical energy which can be recharged with electrical energy after discharge; and
(iii) an energy receiving portion (5) electrically connected to the energy storage (4), which is configured so as to be able to receive energy without contact and to deliver the energy to the energy storage (4) for recharging the energy storage (4); wherein

(I) the energy receiving portion (5) comprises at least one coil (6) extending along a coil axis (SA) and adapted to receive and deliver the energy to the energy storage (4) when passed through by an alternating magnetic field generated by an external charging device, the coil being an air-core coil or comprising a magnetically conductive core (7) located in the coil and extending along the coil axis, wherein

a) the core runs (7) along the coil axis and does not protrude beyond the ends of the coil (6), or
b) the core (7) extends along the coil axis (SA) and protrudes beyond at least one end of the coil (6) to form a field collector (18a, 18b) without increasing a cross-sectional area of the core (7), or
c) the core extends (7) along the coil axis (SA) and protrudes beyond at least one end of the coil to form a field collector (18a, 18b), wherein a cross-sectional area of the core (7) increases, and

(II) the coil (6), when penetrated by the alternating magnetic field, generates a charging current of preferable 2A at maximum rectified by a rectifier, which is fed to the energy store (4) for recharging;

(III) the energy receiving portion has a magnetic field collecting area $A_0$ perpendicular to the coil axis with $A_0 <= 2.5*10^{-3} m^2$ defined by $A_0 = \Phi_{SM}/B_0$, wherein $\Phi_{SM}$ is the magnetic flux passing, as a maximum, through a longitudinal magnetic center within the coil in the direction of the coil axis and $B_0$ is the external mean flux density of the alternating magnetic field over the magnetic field collecting area $A_0$; and

(IV) the electronics assembly (3) is configured to provide information for automatic correction of a deviation between a spatial orientation of a vector of an internal alternating magnetic field of a charging coil of a charging device and the coil axis of the implant, for example to the charging device, thereby allowing the implant (100) to be implantable in any spatial orientation, wherein

the electronics assembly (3) has a memory in which at least one threshold value, e.g. corresponding to a specific charging current level, is stored, and

the electronics assembly (3) is set up to compare the charging current with the threshold value and, when the charging current reaches the threshold value, to supply at least this information to the charging device for automatic correction of the spatial orientation of the coil axis of the implant, immediately and without delay.

7. Implant (100) according to any one of the preceding claims 1 to 6, wherein the core (7) is a magnetically conductive housing of the energy storage on which the coil (6) is wound.

8. Implant (100) according to any one of the preceding claims 1 to 6, wherein the core (7) is a magnetically conductive solid shaft or a magnetically conductive hollow shaft.

9. Implant (100) according to claim 8, wherein the core (7) is the magnetically conductive solid shaft and the magnetically conductive energy storage is located in the direction of the coil axis next to the solid shaft and inside or outside the coil.

10. Implant (100) according to claim 8, wherein the core (7) is the magnetically conductive solid shaft on which the coil is wound, and the magnetically non-conductive energy storage extends radially to the coil axis around the coil.

11. Implant (100) according to claim 8, wherein the core (7) is a magnetically conductive hollow shaft on which the coil is wound, and wherein the energy storage device or devices are arranged within the hollow shaft, preferably with one energy storage device arranged at each end of the hollow shaft.

12. Implant (100) according to claim 8, wherein the core (7) is the magnetically conductive hollow shaft on which the coil is wound, and the magnetically non-conductive energy storage device is located in the direction of the coil axis next to the hollow shaft and inside or outside the coil.

13. Implant (100) according to one of the preceding claims 1 to 12, wherein the coil has W windings and W is $\leq 1000$, preferably W $\leq$ 50, 40, 30, 20, 10.

14. Implant (100) according to one of the preceding claims 1 to 13, wherein the implant generates the charging current as intended at a frequency f of the external alternating magnetic field, wherein f $\leq$ 2 MHz, preferably f $\leq$ 500 kHz, 400 kHz, 300 kHz, 200 kHz, 100 kHz, 50 kHz.

15. The implant (100) according to any one of the preceding claims 1 to 14, wherein the energy receiving portion is constructed such that the longitudinal magnetic center coincides with the longitudinal center of the coil.

16. Implant (100) according to any one of patent claims 1 to 14, wherein

(V) the electronics assembly (3) is set up,

(i) to detect or being signaled by the charging device that the spatial alignment of the alternating magnetic field has been completed; and

(ii) subsequently to send, to the charging device, on the basis of the current charging current, a field change signal which signals to the charging device to increase or decrease a frequency and/or an amplitude of the alternating magnetic field, wherein the electronics assembly (3) signals to the charging device which effects the changes in the frequency and/or the amplitude of the alternating magnetic field entail in order to compensate for intrinsic damping of the alternating magnetic field by the body.

**Revendications**

1. Implant électronique (100) destiné à être implanté dans un corps d'un être vivant et à surveiller une fonction corporelle, en particulier stimulateur cardiaque destiné à surveiller et à contrôler la fonction corporelle, l'implant (100) présentant :

   une section d'électrode (2), qui est destinée, conformément à sa destination, à être fixée à ou disposée sur une partie du corps ; et
   un boîtier, qui comprend un volume $V_G$ dans la plage de $0,5 \le V_G \le 4\,cm^3$, de manière préférée $\le 2\,cm^3$, et qui reçoit les composants suivants de l'implant électronique (100) :

   (i) une électronique (3) reliée à la section d'électrode, qui est configurée pour surveiller au moins la fonction corporelle via la section d'électrode (2) ;
   (ii) un accumulateur d'énergie (4) pour l'alimentation pendant une longue durée de l'électronique (3) en énergie électrique, qui peut être rechargé en énergie électrique après décharge ; et
   (iii) une section de réception d'énergie (5) reliée électriquement à l'accumulateur d'énergie (4), qui est configurée de telle sorte qu'elle peut recevoir de l'énergie sans contact et la délivrer à l'accumulateur d'énergie (4) pour la recharge de l'accumulateur d'énergie (4) ; où

   (I) la section de réception d'énergie (5) présente au moins une bobine (6), qui s'étend le long d'un axe de bobine (SA) et qui est configurée pour recevoir l'énergie et la délivrer à l'accumulateur d'énergie (4) lorsqu'elle est traversée par un champ magnétique alternatif qui est généré par un chargeur externe, la bobine étant une bobine à air ou comprenant un noyau magnétiquement conducteur (7), qui se trouve dans la bobine (6) et s'étend le long de l'axe de bobine (SA), où

   a) le noyau (7) s'étend le long de l'axe de bobine (SA) et ne fait pas saillie au-delà des extrémités de la bobine (6), ou
   b) le noyau (7) s'étend le long de l'axe de bobine (SA) et, pour la formation d'un collecteur de champ (18a, 18b), fait saillie au-delà d'au moins une extrémité de la bobine (6), sans qu'une surface de section transversale du noyau (7) ne soit augmentée, ou
   c) le noyau (7) s'étend le long de l'axe de bobine (SA) et, pour la formation d'un collecteur de champ (18a, 18b), fait saillie au-delà d'au moins une extrémité de la bobine (6), une surface de section transversale du noyau (7) étant augmentée, et

   (II) la bobine (6), lorsqu'elle est traversée par le champ magnétique alternatif, génère un courant de charge, redressé par un redresseur, de préférence d'un maximum de 2 A, qui est fourni à l'accumulateur d'énergie (4) pour la recharge ;
   (III) la section de réception d'énergie (5) présente une surface de captation de champ magnétique $A_0$, disposée perpendiculairement à l'axe de bobine (SA), avec $A_0 <= 2,5*10^{-3}m^2$, qui est définie par $A_0 = \Phi_{SM}/B_0$, où $\Phi_{SM}$ est le flux magnétique qui traverse en tant que maximum un centre longitudinal magnétique situé dans la direction de l'axe de bobine (SA) à l'intérieur de la bobine (6), et $B_0$ est la densité de flux moyenne externe du champ magnétique alternatif sur la surface de captation de champ magnétique $A_0$ ; et
   (IV) l'électronique (3) est configurée pour fournir des informations relatives à la correction automatique d'un écart entre une orientation spatiale d'un vecteur d'un champ magnétique alternatif interne d'une bobine de charge d'un chargeur et l'axe de bobine de l'implant, par exemple au chargeur, de sorte que l'implant (100) est implantable dans n'importe quelle orientation spatiale, où

   selon (IV), l'électronique (3) est configurée pour (i) envoyer au chargeur ou recevoir de celui-ci un signal de démarrage pour démarrer l'ajustement de l'orientation du champ magnétique alternatif, ledit chargeur modifie ensuite l'orientation du champ magnétique alternatif, et (ii) émettre des informations temporelles, qui indiquent à quel moment le courant de charge était approprié pour la recharge, par exemple vers le chargeur, en tant qu'informations de correction.

2. Implant (100) selon la revendication 1, où
   les informations temporelles indiquent au moins un instant ou une plage temporelle auquel/dans laquelle l'amplitude du courant de charge ou son gradient était maximal pour la recharge.

3. Implant (100) selon la revendication 2, où

les informations temporelles indiquent des instants ou des plages temporelles auxquels/dans lesquelles le courant de charge était maximal, et

l'électronique (3) est configurée pour (i) qualifier les instants ou les plages temporelles afin de déterminer si le courant de charge maximal respectif constitue un maximum global ou local, et (ii) émettre au moins l'instant ou la plage temporelle conformément au maximum global ou local, par exemple vers le chargeur, en tant qu'informations de correction.

4. Implant (100) selon l'une des revendications précédentes, où
l'électronique (3) détermine l'intensité du courant de charge à des intervalles réguliers ou irréguliers et, sur cette base, détermine les informations temporelles et les stocke pour l'émission.

5. Implant (100) selon la revendication 1, 2, 3 ou 4, où
(V) l'électronique (3) est configurée pour (i) détecter une fin de l'ajustement spatial de l'orientation du champ magnétique alternatif ou la recevoir comme signal du chargeur, et (ii) émettre ensuite les informations temporelles vers le chargeur.

6. Implant électronique (100) destiné à être implanté dans un corps d'un être vivant et à surveiller une fonction corporelle, en particulier stimulateur cardiaque destiné à surveiller et à contrôler la fonction corporelle, l'implant (100) présentant :

une section d'électrode (2), qui est destinée, conformément à sa destination, à être fixée à ou disposée sur une partie du corps ; et
un boîtier, qui comprend un volume $V_G$ dans la plage de $0,5 \leq V_G \leq 4\,cm^3$, de manière préférée $\leq 2\,cm^3$, et qui reçoit les composants suivants de l'implant électronique (100) :

(i) une électronique (3) reliée à la section d'électrode, qui est configurée pour surveiller au moins la fonction corporelle via la section d'électrode (2) ;
(ii) un accumulateur d'énergie (4) pour l'alimentation pendant une longue durée de l'électronique (3) en énergie électrique, qui peut être rechargé en énergie électrique après décharge ; et
(iii) une section de réception d'énergie (5) reliée électriquement à l'accumulateur d'énergie (4), qui est configurée de telle sorte qu'elle peut recevoir de l'énergie sans contact et la délivrer à l'accumulateur d'énergie (4) pour la recharge de l'accumulateur d'énergie (4) ; où

(I) la section de réception d'énergie (5) présente au moins une bobine (6), qui s'étend le long d'un axe de bobine (SA) et qui est configurée pour recevoir l'énergie et la délivrer à l'accumulateur d'énergie (4) lorsqu'elle est traversée par un champ magnétique alternatif qui est généré par un chargeur externe, la bobine étant une bobine à air ou comprenant un noyau magnétiquement conducteur (7), qui se trouve dans la bobine (6) et s'étend le long de l'axe de bobine (SA), où

a) le noyau (7) s'étend le long de l'axe de bobine (SA) et ne fait pas saillie au-delà des extrémités de la bobine (6), ou
b) le noyau (7) s'étend le long de l'axe de bobine (SA) et, pour la formation d'un collecteur de champ (18a, 18b), fait saillie au-delà d'au moins une extrémité de la bobine (6), sans qu'une surface de section transversale du noyau (7) ne soit augmentée, ou
c) le noyau (7) s'étend le long de l'axe de bobine (SA) et, pour la formation d'un collecteur de champ (18a, 18b), fait saillie au-delà d'au moins une extrémité de la bobine (6), une surface de section transversale du noyau (7) étant augmentée, et

(II) la bobine (6), lorsqu'elle est traversée par le champ magnétique alternatif, génère un courant de charge, redressé par un redresseur, de préférence d'un maximum de 2 A, qui est fourni à l'accumulateur d'énergie (4) pour la recharge ;
(III) la section de réception d'énergie (5) présente une surface de captation de champ magnétique $A_0$, disposée perpendiculairement à l'axe de bobine (SA), avec $A_0 <= 2,5*10^{-3} m^2$, qui est définie par $A_0 = \Phi_{SM}/B_0$, où $\Phi_{SM}$ est le flux magnétique qui traverse en tant que maximum un centre longitudinal magnétique situé dans la direction de l'axe de bobine (SA) à l'intérieur de la bobine (6), et $B_0$ est la densité de flux moyenne externe du champ magnétique alternatif sur la surface de captation de champ magnétique $A_0$ ; et
(IV) l'électronique (3) est configurée pour fournir des informations relatives à la correction automatique d'un écart entre une orientation spatiale d'un vecteur d'un champ magnétique alternatif interne d'une bobine de charge du chargeur et l'axe de bobine de l'implant, par exemple au chargeur, de sorte que l'implant (100) est implantable dans n'importe quelle orientation spatiale, où

l'électronique (3) présente une mémoire dans laquelle au moins une valeur seuil, qui correspond par exemple à une intensité déterminée du courant de charge, est stockée, et

l'électronique (3) est configurée pour comparer le courant de charge avec la valeur seuil, et, lorsque le courant de charge atteint la valeur seuil, fournir au moins cette information relative à la correction automatique de l'orientation spatiale de l'axe de bobine de l'implant au chargeur immédiatement et sans délai.

7. Implant (100) selon l'une des revendications précédentes 1 à 6, où le noyau (7) est un boîtier magnétiquement conducteur de l'accumulateur d'énergie sur lequel la bobine (6) est enroulée.

8. Implant (100) selon l'une des revendications précédentes 1 à 6, où le noyau (7) est un arbre plein magnétiquement conducteur ou un arbre creux magnétiquement conducteur.

9. Implant (100) selon la revendication 8, où le noyau (7) est l'arbre plein magnétiquement conducteur et l'accumulateur d'énergie magnétiquement conducteur se trouve, dans la direction de l'axe de bobine, à côté de l'arbre plein et à l'intérieur ou à l'extérieur de la bobine.

10. Implant (100) selon la revendication 8, où le noyau (7) est l'arbre plein magnétiquement conducteur, sur lequel la bobine est enroulée, et l'accumulateur d'énergie non magnétiquement conducteur s'étend radialement par rapport à l'axe de bobine autour de la bobine.

11. Implant (100) selon la revendication 8, où le noyau (7) est l'arbre creux magnétiquement conducteur, sur lequel la bobine est enroulée, et le(s) accumulateur(s) d'énergie se trouve(nt) à l'intérieur de l'arbre creux, de manière préférée chacun des accumulateurs d'énergie à l'extrémité de l'arbre creux.

12. Implant (100) selon la revendication 8, où le noyau (7) est l'arbre creux magnétiquement conducteur, sur lequel la bobine est enroulée, et l'accumulateur d'énergie non magnétiquement conducteur se trouve, dans la direction de l'axe de bobine, à côté de l'arbre creux et à l'intérieur ou à l'extérieur de la bobine.

13. Implant (100) selon l'une des revendications précédentes 1 à 12, où la bobine présente W enroulements et $W \leq 1\,000$, de manière préférée $W \leq 50, 40, 30, 20, 10$.

14. Implant (100) selon l'une des revendications précédentes 1 à 13, où l'implant génère, conformément à sa destination, le courant de charge à une fréquence f du champ magnétique alternatif externe, où $f \leq 2\,MHz$, de manière préférée f est $\leq 500\,kHz, 400\,kHz, 300\,kHz, 200\,kHz, 100\,kHz, 50\,kHz$.

15. Implant (100) selon l'une des revendications précédentes 1 à 14, où la section de réception d'énergie est construite de telle sorte que le centre longitudinal magnétique coïncide avec le centre longitudinal de la bobine.

16. Implant (100) selon l'une des revendications 1 à 14, où
(V) l'électronique (3) est configurée pour,

(i) détecter une fin de l'ajustement spatial de l'orientation du champ magnétique alternatif ou la recevoir comme signal du chargeur, et
(ii) envoyer ensuite, sur la base du courant de charge momentané, un signal de modification de champ au chargeur, qui signale au chargeur d'augmenter ou de diminuer la fréquence et/ou l'amplitude du champ magnétique alternatif, l'électronique (3) signalant au chargeur quelles conséquences les modifications de la fréquence et/ou de l'amplitude du champ magnétique alternatif entraînent pour la compensation de l'atténuation propre au corps du champ magnétique alternatif.

Volumen bevorzugt 1cm³

Figur 1A

EP 4 596 029 B1

Figur 1B

Figur 1C

Fig. 2A

Figur 2C

EP 4 596 029 B1

Figur 3

Bevorzugte Längsachse
des Patienten

Z- Achse

90°

Drehachse 2

Y

α

b

Achse der Spule des
Ladegerätes in Nullstellung

a

90°

90°

Achse der Spule des
Ladegerätes nach α
→ Örtliches Maximum

Lage der Achse der Spule
des Implantats

Achse der Spule des
Ladegerätes nach Y⊥ α
→Globales Maximum = Endstellung

Drehachse 1
Y-Achse

Geometrischer Ort der beiden
Drehpunkte a, b der Ladespule auf
der Drehachse 2, bei deren
Drehung um die Y-Achse

**Figur 4**

**EP 4 596 029 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20210212586 A1
- US 2017202467 A
- EP 3756726 A2
- EP 4035728 A1